# EUROPEAN PATENT APPLICATION

(11) **EP 4 763 197 A1**
(43) Date of publication of application: **24.06.2026**
(21) Application number: 24221556.4
(22) Date of filing: 19.12.2024
(51) Int. Cl.: A61K 9/50, A61K 9/51, A61K 38/46

(54) **FUNCTIONALIZED ATP-HYDROLYZING ENZYME COMPOSITIONS**

(71) Applicant: MV Biotherapeutics SA, 6500 Bellinzona (CH); Perseo Pharma AG, 4132 Muttenz (CH)
(72) Inventor: GRASSI, Fabio, 6500 Bellinzona (CH); BRIAND, Manon, 5408 Ennetbaden (CH); DUDAL, Yves Victor René, 4103 Bottmingen (CH); SPECIOSO, Gabriele, 4303 Kaiseraugst (CH)
(74) Representative: Latscha Schöllhorn Partner AG

(57) **Abstract**

The present invention relates to a composition comprising a solid carrier, a protein ATP-hydrolyzing enzyme or a fragment thereof immobilized on the surface of the solid carrier, a protective layer to protect the protein ATP-hydrolyzing enzyme or a fragment thereof by embedding the protein ATP-hydrolyzing enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group. The present invention also relates to methods of producing said composition and uses of the composition for the prevention, delay of progression or treatment of dysbiosis or a dysbiosis-related disease. The present invention also relates to a pharmaceutical combination comprising (i) said composition; and (ii) an immune checkpoint modulator and uses thereof for the prevention, delay of progression or treatment of cancer and/or for use in adoptive (T) cell therapy.

## Description

### The field of the invention

The present invention relates to a composition comprising a solid carrier, an ATP-hydrolyzing enzyme or a fragment thereof immobilized on the surface of the solid carrier, a protective layer to protect the ATP-hydrolyzing enzyme or a fragment thereof by embedding the ATP-hydrolyzing enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group. The present invention also relates to methods of producing said composition and uses of the composition for the prevention, delay of progression or treatment of dysbiosis or a dysbiosis-related disease. The present invention also relates to a pharmaceutical combination comprising (i) said composition; and (ii) an immune checkpoint modulator and uses thereof for the prevention, delay of progression or treatment of cancer and/or for use in adoptive (T) cell therapy.

### Background of the invention

The human gastrointestinal (GI) tract is a complex ecological niche, in which all the three domains of life (Archaea, Bacteria and Eukarya) and Viruses co-exist in close association with the host. This complex microbial community, referred to as the gut microbiota, has co-evolved with the host in a mutualistic relationship that influences many physiological functions such as energy harvesting, development and function of the immune system. The subtle equilibrium between the gut microbiota and the host is a key element in human health. In fact, alterations in the composition of the microbial community structure, termed dysbiosis, have been associated to an increasing number of medical conditions such as metabolic disorders (e.g. diabetes, obesity) (Holmes, E., Loo, R.L., Stamler, J., Bictash, M., Yap, I.K., Chan, Q., Ebbels, T., De Iorio, M., Brown, I.J., Veselkov, K.A., et al. (2008). Human metabolic phenotype diversity and its association with diet and blood pressure. Nature 453, 396-400; Larsen, N., Vogensen, F.K., van den Berg, F.W., Nielsen, D.S., Andreasen, A.S., Pedersen, B.K., Al-Soud, W.A., Sorensen, S.J., Hansen, L.H., and Jakobsen, M. (2010). Gut microbiota in human adults with type 2 diabetes differs from non-diabetic adults. PLoS One 5, e9085; Tumbaugh, P.J., Hamady, M., Yatsunenko, T., Cantarel, B.L., Duncan, A., Ley, R.E., Sogin, M.L., Jones, W.J., Roe, B.A., Affourtit, J.P., et al. (2009). A core gut microbiome in obese and lean twins. Nature 457, 480-484), blood pressure alteration and heart disease (Blaser, M.J. (2006). Who are we? Indigenous microbes and the ecology of human diseases. EMBO Rep 7, 956-960), and autoimmunity (Dicksved, J., Halfvarson, J., Rosenquist, M., Jarnerot, G., Tysk, C., Apajalahti, J., Engstrand, L., and Jansson, J.K. (2008). Molecular analysis of the gut microbiota of identical twins with Crohn's disease. ISME J 2, 716-727). Various other diseases are known to be related to dysbiosis including inflammatory diseases, gastrointestinal tract-related disorders, metabolic disorders, CNS-related disorders, cancers and autoimmune diseases like inflammatory bowel disease (IBD) (Sartor R.B., Wu G.D. Gastroenterology, 152: 327-339.e4 (2017); Knox N., Forbes J., Peterson C-L, Van Domselaar G., Bernstein C. Am J Gastroenterol. 114: 1051-1070 (2019); Illiano P., Brambilla R., Parolini C. FEBS J. 287: 833-855 (2020); Islam M.M., Mahbub N.U., Hong S.T., Chung H.J.. Front Cell Infect Microbiol. 14: 1291148 (2024).

In summary, dysbiosis of human microbiota is associated with a number of diseases. Therefore, several options have been studied to prevent or treat dysbiosis or dysbiosis-related diseases. For a long time, antibiotics were administered to select which bacteria to preserve in the intestinal ecosystem (Sanders, W. E., Jr., Sanders, C. C.: Modification of normal flora by antibiotics: effects on individuals and the environment. In: Koot, R. K., Sande, M. A. (ed.): New dimensions in antimicrobial therapy. Churchill Livingstone, New York, 1984, p. 217-241). However, this strategy is now being actively avoided because of its selection pressure and the risks of emergence of antibiotic-resistant bacteria. Other strategies include fecal microbiota transplants (FMTs), which are currently used to treat patients with Clostridium difficile infections, who have proved resistant to other therapies (Smith MB, Kelly C, Alm EJ (February 2014). "Policy: How to regulate faecal transplants". Nature. 506 (7488): 290-291. doi:10.1038/506290a). However, this therapy is still investigational and there are concerns in particular because the process is not sterile and contaminations can pass from donor to patient. In view thereof, probiotics and prebiotics recently emerged as promising tools for the treatment of dysbiosis. However, under certain circumstances, their efficacy may be limited. Thus there is a need to provide effective treatments for dysbiosis or a dysbiosis-related disease.

### Summary of the invention

The present invention provides a composition comprising a solid carrier, an ATP-hydrolyzing enzyme or a fragment thereof immobilized on the surface of the solid carrier, a protective layer to protect the ATP-hydrolyzing enzyme or a fragment thereof by embedding the ATP-hydrolyzing enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group.

The present invention provides also a method of producing said composition comprising a solid carrier, an ATP-hydrolyzing enzyme or a fragment thereof immobilized on the surface of the solid carrier, a protective layer to protect the ATP-hydrolyzing enzyme or a fragment thereof by embedding the ATP-hydrolyzing enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group, the method comprising the following steps:
(a) providing a solid carrier;
(b) immobilizing an ATP-hydrolyzing enzyme or a fragment thereof on the solid carrier;
(c) forming a protective layer on the surface of the solid carrier to protect the ATP-hydrolyzing enzyme or a fragment thereof immobilized on the solid carrier;
(d) immobilizing a functional constituent on the surface of the protective layer, wherein the functional constituent immobilized on the surface of the protective layer is a polymer comprising repeat units wherein each repeat unit comprises at least one amino group and/or at least one thiol group.

The present invention also provides a method for the prevention, delay of progression or treatment of dysbiosis or a dysbiosis-related disease using the composition of the present invention.

The present invention also provides a pharmaceutical combination comprising (i) said composition; and (ii) an immune checkpoint modulator and uses thereof for the prevention, delay of progression or treatment of cancer and/or for use in adoptive (T) cell therapy.

It has been surprisingly found by the inventors of the present application that compositions as provided by the present invention if applied therapeutically demonstrate at the mucosal level a prolonged immunomodulatory activity and a surprising efficacy at low dosage in controlling the pathological consequences of colonic inflammation.

### Brief description of the figures

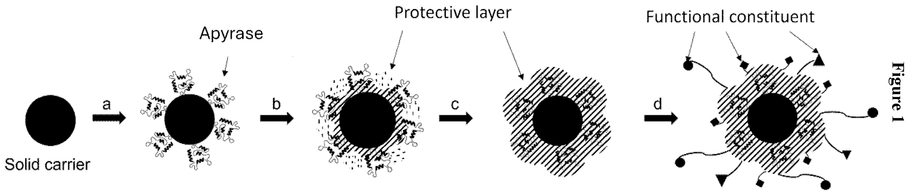

**Figure 1****)** shows a schematic representation of the process for the production of the composition of the invention: a) Apyrase or fragment thereof is immobilized on the solid carrier; b) and c) a protective layer grows around the immobilized apyrase or fragment thereof embedding the immobilized apyrase or fragment thereof; and d) a functional constituent is immobilized on the surface of the protective layer
**Figure 2****)** shows Scanning Electron Microscopy of apyrase-based SNPs (SNP-Apyrase-AT). A) SEM micrograph of SNP-Apyrase-AT without Chitosan. B) SEM micrograph of SNP-Apyrase-AT functionalized with Chitosan (SNP-Apyrase-AT-Chitosan).
**Figure 3****)** shows size distribution of SNP-apyrase-AT (A) and SNP-apyrase-AT-Chitosan (B) diameters.
**Figure 4****)** shows apyrase immobilization efficiency: amount of enzyme immobilized in SNP-apyrase-AT (A) and SNP-apyrase-AT-Chitosan (B) compared with the total amount used for the immobilization process
**Figure 5****)** shows ATPase activity per gram of SNP-apyrase-AT (A) and SNP-apyrase-AT-Chitosan (B).
**Figure 6**) shows treatment regimen of mice with DSS 2%, recombinant apyrase (22 mU) and SNP-apyrase-AT-Chitosan (2.2 mU).
**Figure 7**) shows weight loss of treated mice.
**Figure 8**) shows permeability to FITC-dextran of treated mice.
**Figure 9**) shows reduced colon length of treated mice.

### Detailed description of the invention

The present invention relates to a composition comprising a solid carrier, an ATP-hydrolyzing enzyme or a fragment thereof immobilized on the surface of the solid carrier, a protective layer to protect the ATP-hydrolyzing enzyme or a fragment thereof by embedding the ATP-hydrolyzing enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group.

For the purposes of interpreting this specification, the following definitions will apply and whenever appropriate, terms used in the singular will also include the plural and *vice versa.* It is to be understood that the terminology used herein is for the purpose of describing particular embodiments only and is not intended to be limiting.

Features, integers, characteristics, compounds described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments.

The term "comprise" and variations thereof, such as, "comprises" and "comprising" is generally used in the sense of include, that is, as "including, but not limited to", that is to say permitting the presence of one or more features or components.

The singular forms "a," "an," and "the" include plural referents unless the content clearly dictates otherwise.

The term "about" refers to a range of values ± 10% of a specified value. For example, the phrase "about 200" includes ± 10% of 200, or from 180 to 220.

The term "solid carrier" as used herein refers usually to a particle. Preferably the solid carrier is a monodisperse particle or a polydisperse particle, more preferably a monodisperse particle. The solid carrier usually comprises organic particles, inorganic particles, organic-inorganic particles, self-assembling organic particles, silica particles, gold particles, titanium particles and is preferably a silica particle, more preferably a silica nanoparticle (SNP). The particle size of the solid carrier is usually between 1 nm and 1000 µm, preferably between 10 nm and 100 µm, particularly about 50 nm.

The term "linker" or "cross-linker" which are used synonymously herein refers to any linking reagents containing groups, which are capable of binding to specific functional groups (e.g. primary amines, sulfhydryls, etc.). A linker in the context of the present invention usually connects the surface of the solid carrier with the ATP-hydrolyzing enzyme. For example, a linker may be immobilized on the surface of the solid carrier e.g. on the silica surface as a carrier material and then the ATP-hydrolyzing enzyme may be bound to an unoccupied binding-site of the linker. Alternatively, the linker may firstly bind to the ATP-hydrolyzing enzyme and then the linker bound to the ATP-hydrolyzing enzyme may bind with its unoccupied binding-site to the solid carrier. Various types of linkers are known in the art, including but not limited to straight or branched-chain carbon linkers, heterocyclic carbon linkers, peptide linkers, polyether linkers, and linkers that are known in the art as tags.

The term "protective layer" as used herein refers to a layer for protecting the functional properties of the ATP-hydrolyzing enzyme or fragment immobilized on the surface of the solid carrier. The protective layer of the present invention is usually built with building blocks at least part of which are monomers capable of interacting with both each other usually by covalent binding and the immobilized ATP-hydrolyzing enzyme usually by non-covalent binding. The protective layer is formed on the surface of the solid carrier to protect the ATP-hydrolyzing enzyme or the fragment thereof immobilized on the solid carrier. The protective layers are usually homogeneous layers where at least 50%, preferably at least 70%, more preferably at least 90% of the ATP-hydrolyzing enzyme or fragment therof are embedded in the protective layer.

The term "ATP-hydrolyzing enzyme or a fragment thereof" refers to any enzyme which catalyzes the hydrolysis of ATP to ADP, ATP to AMP and/or ADP to AMP. Such enzymes include but are not limited to apyrase, ATPase, ATP-diphosphatase, adenosine diphosphatase, ADPase, ATP-diphosphohydrolase and CD39 (Ectonucleoside triphosphate diphosphohydrolase 1, ENTPD1). In the context of the present invention, any ATP-hydrolyzing enzyme may be used. Examples of ATP-hydrolyzing enzymes include soluble bacterial (e.g., *Shigella flexneri*) and soluble potato apyrase as well as (engineered) soluble CD39.

An ATP-hydrolyzing enzyme or a fragment thereof includes naturally occurring ATP-hydrolyzing enzymes or a fragment thereof and also includes artificially engineered ATP-hydrolyzing enzymes or a fragment thereof. Artificially engineered ATP-hydrolyzing enzymes or a fragment thereof are e.g. variants or functionally active fragments of the ATP-hydrolyzing enzyme. The terms "fragment of a ATP-hydrolyzing enzyme", "fragment thereof' in relation to the ATP-hydrolyzing enzyme and "functionally active fragment of the ATP-hydrolyzing enzyme" are thus used synonymously herein. By "variants" or "functionally active fragments thereof' in relation to the ATP-hydrolyzing enzyme of the present invention is meant that the fragment or variant (such as an analogue, derivative or mutant) is capable of exercising the same physiological function as the ATP-hydrolyzing enzyme. Such variants include naturally occurring allelic variants and non-naturally occurring variants. Additions, deletions, substitutions and derivatizations of one or more of the amino acids are contemplated so long as the modifications do not result in loss of functional activity of the fragment or variant. Preferably the functionally active fragment or variant has at least about 80% sequence identity more preferably at least about 90% sequence identity, even more preferably at least about 95% sequence identity, most preferably at least about 98% sequence identity to the relevant part of the ATP-hydrolyzing enzyme. A fragment of a ATP-hydrolyzing enzyme as defined herein does usually have the same functional properties as the ATP-hydrolyzing enzyme from which it is derived. A fragment of an ATP-hydrolyzing enzyme, in particular a fragment of apyrase contains usually between 100 and 500 amino acids, preferably between 200 and 375 amino acids, more preferably between 200 and 250 amino acids.

The term "partially embedded ATP-hydrolyzing enzyme" as used herein shall mean that the ATP-hydrolyzing enzyme is not fully covered by the protective layer, thus, the ATP-hydrolyzing enzyme is not fully embedded in the protective layer. In one embodiment less than 50% of the ATP-hydrolyzing enzyme of interest are covered by the protective layer, though typically more at least 70% will be covered, thus improving protection of the ATP-hydrolyzing enzyme. In a preferred embodiment, at least 70%, more preferably at least 80%, even more preferably at least 90%, most preferably at least 95% of the ATP-hydrolyzing enzyme of interest is covered by the protective layer. In another preferred embodiment, around 70% to around 95%, more preferrably around 80% to around 95%, even more preferably around 90% to around 95%, most preferably around 90% to around 95, 96, 97, 98 or 99 % of the ATP-hydrolyzing enzyme of interest are covered by the protective layer. In a particularly preferred embodiment, around 70%, particularly around 80%, more particularly around 90%, most particularly around 95% of the ATP-hydrolyzing enzyme of interest is covered by the protective layer. In a more particularly preferred embodiment, around 70%, particularly around 80%, more particularly around 90%, most particularly around 95% of the ATP-hydrolyzing enzyme of interest is covered by the protective layer, wherein the active site is not covered.

The term "fully embedded ATP-hydrolyzing enzyme" as used herein shall mean that the ATP-hydrolyzing enzyme of interest according to the invention is fully, i.e. 100% covered by the protective layer, i.e. that also the active site is covered.

The term "at least partially embedded ATP-hydrolyzing enzyme" as used herein shall mean that the ATP-hydrolyzing enzyme is at least partially embedded and may be fully embedded by the protective layer. Thus "at least partially embedded ATP-hydrolyzing enzyme" means that the protective layer covers from about 30% and 100% of the ATP-hydrolyzing enzyme or a fragment therof, preferably from about 50% to about 100%, more preferably from about 80% to about 100%, even more preferably from about 90% to about 100%, most preferably from about 95% to about 100 %, wherein the active site is preferably covered.

The term "functional constitutent" as used herein refers to a constituent which after being immobilized to the surface of the protective layer retains its characteristic, functional property. A functional constituent in the sense of the present invention is a polymer comprising repeat units wherein each repeat unit comprises at least one amino group and/or at least one thiol group.

The term "polymer comprising repeat units wherein each repeat unit comprises at least one amino group" as used herein refers to a polymer comprising a number of repeat units (monomers), whererin each repeat unit comprises at least one amino group. A preferred polymer comprises a number of repeat units (monomers), whererin each repeat unit contains one amino group, in particular one primary amino group.

The term "polymer comprising repeat units wherein each repeat unit comprises at least one thiol group" as used herein refers to a polymer comprising a number of repeat units (monomers), whererin each repeat unit comprises at least one thiol. A preferred polymer comprises a number of repeat units (monomers), whererin each repeat unit contains one thiol group.

The term "polycarbophil-cysteine conjugates" as used herein refers to conjugates which comprise cysteine covalently attached to polycarbophil. Such conjugates can be produced as referred in e.g. Bernkop-Schnurch and Thaler, 2000, Journal of Pharmaceutical Sciences 89(7):901-9.

The term "polylysine" as used herein refers to α-polylysine and or ε-polylysine (ε-poly-L-lysine, EPL), preferably ε-polylysine. α-polylysine is a synthetic polymer, which can be composed of either L-lysine or D-lysine. ε-polylysine (ε-poly-L-lysine, EPL) is typically produced as a homopolypeptide of approximately 25-30 L-lysine residues.

The term "polycysteine" as used herein can be composed of either L-cysteine or D-cysteine and is preferably composed of L-cysteine and comprises preferably between 2 and 30 cysteine residues, more preferably between 2 and 5 cysteine residues.

The term "polyglucosamin" as used herein refers to linear amino-polysaccharides composed of D-glucosamine and N-acetyl-D-glucosamine units linked by (1-4) glycosidic bonds. Polyglucosamine contains free amine (-NH2) groups and may be characterized by the proportion of N-acetyl-D-glucosamine units and D-glucosamine units, which is expressed as the degree of deacetylation (DDA) of the fully acetylated polymer chitin. A preferred polyglucosamin of the present invention is selected from the group consisting of chitin, chitosan, polyglucosaminoglycans, chondroitin, heparin, keratan and dermatan or a derivative thereof. Most preferred is a chitosan or a derivative thereof.

The term "chitosan or a derivative thereof' as used herein refers to a chitosan or chitosan derivative thereof including a salt thereof which has preferably a molecular weight of 2 000 Da or more, preferably in the range 25 000 - 2 000 000 Da and more preferably about 50 000 - 350 000 Da, most preferably about 50 000 - 190 000 Da or 190 000 - 310 000 Da. The term "derivative" in relation to chitosan includes ester, ether or other derivatives formed by reaction of acyl or alkyl groups with the OH groups. Examples are O-alkyl ethers of chitosan, O-acyl esters of chitosan. Suitable derivatives are given e.g. in G.A.E. Roberts, Chitin Chemistry, MacMillan Press Ltd, London, 1992. Suitable salts of chitosan include nitrates, phosphates, sulphates, xanthates, hydrochlorides, glutamates, lactates, acetates.

The term "dysbiosis", as used herein, refers to an abnormal microbiome structure, which affects the taxonomical composition as well as the metagenomic function of the microbial community. Accordingly, dysbiosis is an imbalance in the composition of microbiota, in particular of human microbiota. As used herein, the term "microbiota" refers to commensal microorganisms found in and on all multicellular organisms studied to date from plants to animals. In particular, microbiota have been found to be crucial for immunologic, hormonal and metabolic homeostasis of their host. In particular, microbiota are non-pathogenic. In other words, microbiota (in their normal, balanced composition) are usually not (capable of) causing a disease in the host and/or they are not harmful to the host. Accordingly, the interaction between microbiota and their host is usually commensal or symbiotic. Microbiota include bacteria, archaea, protists, fungi, viruses and phages. Anatomically, microbiota reside on or within any of a number of tissues and biofluids, including the gastrointestinal tract (GI), in particular the gut (and the oral cavity, in particular the oral mucosa), skin, conjunctiva, mammary glands, vagina, placenta, seminal fluid, uterus, ovarian follicles, lung and saliva. Dysbiosis is most commonly reported as a condition in the gastrointestinal tract, for example during small intestinal bacterial overgrowth (SIBO) or small intestinal fungal overgrowth (SIFO). In some embodiments, the microbiota are gastrointestinal tract (GI) microbiota and the dysbiosis is, thus, gastrointestinal dysbiosis. GI microbiota may be selected from gut microbiota and oral cavity microbiota and, therefore, GI dysbiosis may be selected from gut dysbiosis and oral cavity dysbiosis. Intestinal dysbiosis is the disturbance of the normal balance of microbiota species in the intestine. Symptoms of intestinal dysbiosis include upset stomach, nausea, constipation, diarrhea, and bloating.

Dysbiosis can be induced by various factors including external factors (such as administration of antibiotics or chemotherapeutic agents, some substances introduced in diet, physical and psychological stress) and host-related factors. Major causes of dysbiosis include dietary disorders (a hyperprotein hyperlipid diet, rich in sugars and low in fiber; food allergies; malabsorption and impaired digestion of carbohydrates), poor digestive secretions, stress, antibiotic/pharmacological therapy, weakened immune functions, malabsorption, intestinal infections and alterations of the pH in the gastrointestinal tract. In some embodiments, the dysbiosis is induced by antibiotics. In other embodiments, the dysbiosis is induced by chemotherapeutic agents. In some embodiments, dysbiosis may be induced by a dysbiosis-inducing agent as described herein (such as an antibiotic agent or a chemotherapeutic agent), by a diet or by maternal dysbiosis.

Maternal dysbiosis may have a major impact on the offspring. Accordingly, the composition as described herein may also be used for the treatment of newborns and infants of mothers suffering from dysbiosis. In some embodiments, this includes newborns and infants of up to one year of age (for human infants). Accordingly, the composition may be administered to newborns or infants up to one year. In some embodiments, this includes newborns and infants during (and up to four weeks after the end of) breast feeding. Accordingly, the composition may be administered to newborns or infants during (and up to four weeks after the end of) breast feeding.

In summary, "dysbiosis" refers to the disruption of the balance of the microbiota and, consequently, its normal functioning. This results in the selective suppression of some species in the microbiota leading to unregulated production of microorganism-derived products or metabolites that can become dangerous for the host, to the point of causing various disorders locally, systemically or even in more distant organs. Accordingly, dysbiosis is an abnormal microbial ecological state that is causally linked to the manifestation of various diseases.

In dysbiosis, normally dominating microbiota species become underrepresented, while normally outcompeted or contained species may increase to fill the void. As normally dominating microbiota species are usually benign or beneficial and carry out a series of helpful and necessary functions, such as aiding in digestion and providing protection from pathogenic microbes, the selective suppression of beneficial microbiota species in dysbiosis leads to unregulated production of microorganism-derived products or metabolites that can become dangerous for the host, to the point of causing various disorders locally, systemically or even in more distant organs. Accordingly, dysbiosis can trigger the onset of chronic disease in various ways. For example, pathogens and their functions can be acquired or opportunistically overgrow in dysbiosis, which results in infectious diseases such as cholera or streptococcal pharyngitis, but which can also lead to chronic inflammation. Moreover, health-protective bacteria and their functions may be lost or suppressed, which then promotes the onset of diseases, in particular chronic diseases such as inflammatory bowel disease (IBD), urinary stone disease (USD), obesity, and others.

Various diseases are known to be related to dysbiosis including inflammatory diseases, gastrointestinal tract-related disorders, metabolic disorders, CNS-related disorders, cancers and autoimmune diseases. Accordingly, the dysbiosis-related disease may be selected from inflammatory diseases, infectious diseases, gastrointestinal tract-related disorders, metabolic disorders, CNS-related disorders, cancers and autoimmune diseases.

Non-limiting examples of inflammatory diseases include pancreatitis, gingivitis, periodontitis, inflammatory bowel disease (IBD), Crohn's disease (CD), ulcerative colitis (UC), gastritis, enteritis, esophagitis, diverticulitis, rheumatoid arthritis and infectious colitis. Non-limiting examples of infectious diseases include a gastrointestinal infection, a respiratory infection, a kidney infection, and infections with specific pathogens, such as Clostridioides difficile infection and Citrobacter rodentium infection. Non-limiting examples of gastrointestinal tract-related disorders and metabolic disorders include inflammatory bowel disease (IBD), Crohn's disease (CD), ulcerative colitis (UC), gastritis, enteritis, esophagitis, gastroesophageal reflux disease (GERD), celiac disease, ulcer, irritable bowel syndrome, obesity, diabetes, and metabolic syndrome. In general, non-limiting examples of diseases induced by or associated with dysbiosis include inflammatory bowel disease, irritable bowel syndrome, obesity, diabetes, metabolic syndrome, coeliac disease, colorectal cancer, Clostridioides difficile infection, autism spectrum disorder, urinary stone disease (USD), lupus erythematosus, rheumatoid arthritis, systemic sclerosis, Sjögren's syndrome, anti-phospholipid syndrome, cardiovascular syndrome, allergy, and asthma. Accordingly, the dysbiosis-related disease may be selected from inflammatory bowel disease, irritable bowel syndrome, obesity, diabetes, metabolic syndrome, coeliac disease, colorectal cancer, Clostridioides difficile infection, autism spectrum disorder, urinary stone disease (USD), lupus erythematosus, rheumatoid arthritis, systemic sclerosis, Sjögren's syndrome, anti-phospholipid syndrome, cardiovascular syndrome, allergy, and asthma. In some embodiments, the dysbiosis-related disease is irritable bowel syndrome (IBS) or inflammatory bowel disease (IBD), such as Crohn's disease (CD) and/or ulcerative colitis (UC). IBD is a group of inflammatory conditions of the colon and small intestine, which includes Crohn's disease and ulcerative colitis. IBD-affected individuals have been found to have 30-50 percent reduced biodiversity of commensal bacteria, such as decreases in Firmicutes (namely Lachnospiraceae) and Bacteroidetes.

The term "immune checkpoint modulator" (also referred to as "checkpoint modulator") as used herein refers to a molecule or to a compound that modulates (e.g., totally or partially reduces, inhibits, interferes with, activates, stimulates, increases, reinforces or supports) the function of one or more (immune) checkpoint molecules. In other words, an "immune checkpoint modulator" is a modulator of an immune checkpoint molecule. Thus, an immune checkpoint modulator may be an "immune checkpoint inhibitor" (also referred to as "checkpoint inhibitor" or "inhibitor") or an "immune checkpoint activator" (also referred to as "checkpoint activator" or "activator"). An "immune checkpoint inhibitor" (also referred to as "checkpoint inhibitor" or "inhibitor") totally or partially reduces, inhibits, interferes with, or negatively modulates the function of one or more checkpoint molecules. An "immune checkpoint activator" (also referred to as "checkpoint activator" or "activator") totally or partially activates, stimulates, increases, reinforces, supports or positively modulates the function of one or more checkpoint molecules. Immune checkpoint modulators are typically able to modulate (i) self-tolerance and/or (ii) the amplitude and/or the duration of the immune response. Preferably, the immune checkpoint modulator used according to the present invention modulates the function of one or more human checkpoint molecules and is, thus, a "human checkpoint modulator". Preferably, the immune checkpoint modulator is an activator or an inhibitor of one or more immune checkpoint point molecule(s) selected from CD27, CD28, CD40, CD122, CD137, OX40, GITR, ICOS, A2AR, B7-H3, B7-H4, BTLA (CD272), CTLA-4, IDO, KIR, LAG3, PD-1, PD-L1, PD-L2, TIM-3, VISTA, CEACAM1, GARP, PS, CSF 1R, CD94/NKG2A, TDO, GITR, TNFR, TIGIT and/or FasR/DcR3; or an activator or an inhibitor of one or more ligands thereof. Immune checkpoint modulator useful for the present invention are described e.g. in WO2021245071 A1, which is herein incorporated by reference.

In a first aspect the present invention provides a composition comprising a solid carrier, an ATP-hydrolyzing enzyme or a fragment thereof immobilized on the surface of the solid carrier, a protective layer to protect the ATP-hydrolyzing enzyme or a fragment thereof by embedding the ATP-hydrolyzing enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group.

The ATP-hydrolyzing enzyme or a fragment thereof can be immobilized on the surface of the solid carrier by non-covalent binding or covalent binding. Non-covalent binding includes p-p (aromatic) interactions, van der Waals interactions, H-bonding interactions, and electrostatic interactions like e.g. ionic interactions. Preferably, the ATP-hydrolyzing enzyme or fragment thereof is immobilized on the surface of the solid carrier by covalent binding or by covalent binding via a linker.

A solution of a ATP-hydrolyzing enzyme or a fragment thereof usually comprises the protein or a fragment thereof in a buffer solution. Buffers which can be used are usually phosphate, chloride, MES, MOPS, HEPES, PIPES, ACES or mixtures thereof. The solution may additionally contain non-ionic surfactants as described herein. A solution of a ATP-hydrolyzing enzyme or a fragment thereof can be prepared by e.g. dissolving the ATP-hydrolyzing enzyme or a fragment thereof in water to reconstitute the stock buffer of ATP-hydrolyzing enzyme or a fragment thereof.

In one embodiment the solid carrier is selected from the group of organic particles, inorganic particles, organic-inorganic particles, self-assembling organic particles, silica particles, gold particles, titanium particles and is preferably a silica particle, more preferably a silica nanoparticle (SNP). The particle size is usually measured by measuring the diameter of the particles and is usually between 1 nm and 1000 nm, preferably between 10 nm and 100 nm, particularly about 50 nm. In case the solid carrier is a monodisperse particle, the size is usually between 1 nm and 1000 nm, preferably between 10 nm and 100 nm, particularly about 50 nm. In case the solid carrier is a polydisperse particle, the size is usually between1 nm and 1000 µm, preferably between 10 nm and 100 µm, particularly between 50 nm and 50 µm. In one embodiment the composition comprises a solid carrier wherein the solid carrier comprises at least 15%, preferably at least 20%, in particular between 15% and 30%, more particular between 20% and 30 % immobilized ATP-hydrolyzing enzyme or fragment thereof per dry weight of the solid carrier.

Usually monodisperse particles or polydisperse particles, preferably monodisperse particles are used as solid carrier in the present invention. In a preferred embodiment the monodisperse particles are spherical monodisperse particles. In a further preferred embodiment, the polydisperse particles are non-spherical polydisperse particles.

The solid carrier is usually provided in suspension. Suspension of the solid carrier can be e.g. in water, buffer or non-ionic surfactants or mixtures thereof, preferably in mixtures of water and non-ionic surfactants. Non-ionic surfactants used are usually polysorbate 80 (PS80). Buffers which can be used in the method of the present invention are phosphate, piperazine-N,N'-bis(2-ethanesulfonic acid), 2-Hydroxy-3-morpholinopropanesulfonic acid, N,N-bis[2-hydroxyethyl]-2-aminoethanesulfonic acid), (3-(N-morpholino)propanesulfonic acid), 2-[[1,3-dihydroxy-2-(hydroxymethyl)propan-2-yl]amino]ethanesulfonic acid, 4-(2-hydroxyethyl)-1-piperazineethanesulfonic acid), 3-(N,N-Bis[2-hydroxyethyl]amino)-2-hydroxypropanesulfonic acid, N,N-Bis(2-hydroxyethyl)-3-amino-2-hydroxypropanesulfonic acid, N-[Tris(hydroxymethyl)methyl]glycine, Diglycine, 4-(2-Hydroxyethyl)-1-piperazinepropanesulfonic acid, N,N-Bis(2-hydroxyethyl)glycine, N-[Tris(hydroxymethyl)methyl]-3-aminopropanesulfonic acid, N-(1,1-Dimethyl-2-hydroxyethyl)-3-amino-2-hydroxypropanesulfonic acid.

In one embodiment the surface of the solid carrier is modified to introduce a molecule or functional chemical group as anchoring point i.e. as anchoring point for the ATP-hydrolyzing enzyme or for the linker connecting the ATP-hydrolyzing enzyme to the solid carrier. Preferably, said anchoring point is an amine functional chemical group or moiety. As a non-limiting example, an amino-modified surface of the solid carrier e.g. an amino-modified silica surface may be used as modified solid carrier. Such an amino-modified surface of the solid carrier may be obtained by reacting a solid carrier having a silica surface with an amino silane, e.g. with APTES. Thus in a preferred embodiment, the solid carrier is a solid carrier having a silica surface with an amino-modified surface, more preferably a solid carrier obtained by reacting the solid carrier having a silica surface with an amino silane, e.g. with APTES. Such a modified carrier may form an amide linkage between the ATP-hydrolyzing enzyme and the amine group at the surface of the carrier material or an amide linkage between the linker and the amine group at the surface of the carrier material. In one embodiment the introduced molecule or functional chemical group as anchoring point is homogeneously distributed on the surface of the solid carrier.

In some embodiments the protective layer has a defined thickness of about 1 to about 200 nm, usually 1 to about 100 nm, preferably about 1 to about 50nm, more preferably about 1 to about 25 nm, even more preferably about 1 to about 20 nm, in particular about 1 to about 15 nm. The most preferred defined thickness is about 1 to about 10 nm. In some embodiments the layer has a defined thickness of about 5 to about 100 nm, preferably about 5 to about 50 nm, more preferably about 5 to about 25 nm, even more preferably about 5 to about 20 nm, in particular about 5 to about 15 nm. The most preferred defined thickness is about 5 to about 10 nm. The protective layer is usually porous and the pore size is usually between 1 and 20 nm, preferably between 1 and 5 nm".. The protective layer thickness can be measured, by using a microscope such as scanning electron microscope (SEM), transmission electron microscopy (TEM), scanning probe microscopy (SPM), light scattering methods or by ellipsometry.

In one embodiment, the ATP-hydrolyzing enzyme or fragment thereof is partially embedded by the protective layer. In a preferred embodiment the ATP-hydrolyzing enzyme or fragment thereof is at least partially embedded by the protective layer. In a more preferred embodiment the ATP-hydrolyzing enzyme or fragment thereof is fully embedded by the protective layer. In one embodiment, the protective layer embeds the solid carrier and embeds the ATP-hydrolyzing enzyme or fragment thereof immobilized on the surface of the solid carrier. In one embodiment, the functional constituent immobilized on the surface of the protective layer, is not embedded by the protective layer. Preferably, the protective layer fully embeds the solid carrier and fully embeds the ATP-hydrolyzing enzyme or fragment thereof immobilized on the surface of the solid carrier. More preferably, the protective layer fully embeds the solid carrier and fully embeds the ATP-hydrolyzing enzyme or fragment thereof immobilized on the surface of the solid carrier and the functional constituent immobilized on the surface of the protective layer is not embedded by the protective layer. If the protective layer fully embeds the solid carrier and fully embeds the ATP-hydrolyzing enzyme or fragment thereof immobilized on the surface of the solid carrier, the ATP-hydrolyzing enzyme or fragment thereof is fully, i.e. 100% covered by the protective layer, i.e. that also the active site is covered and the solid carrier is fully, i.e. 100% covered by the protective layer.

In one embodiment the ATP-hydrolyzing enzyme is an ATP-diphosphatase or a fragment thereof. In a preferred embodiment the ATP-hydrolyzing enzyme is apyrase or a fragment thereof. Apyrases are ATP-diphosphohydrolases that catalyze the sequential hydrolysis of ATP to ADP and ADP to AMP releasing inorganic phosphate. In particular, apyrases can also act on ADP and other nucleoside triphosphates and diphosphates in addition to ATP. Apyrase can be found in various eukaryotes in membrane bound and/or secreted soluble forms. For example, the apyrase may be *Shigella flexneri* apyrase or Solanum tuberosum (potato) apyrase. Moreover, the apyrase may be a variant of a naturally occurring apyrase. Preferably, the apyrase comprises the amino acid sequence of SEQ ID NO: 1 or SEQ ID NO: 2, more preferably the amino acid sequence of SEQ ID NO: 1. In variants of SEQ ID NO: 1 R192 must be maintained to ensure functionality. The ATP-hydrolyzing enzyme may be obtained by any means. Preferably, the ATP-hydrolyzing enzyme is recombinantly produced. Preferably, the ATP-hydrolyzing enzyme is recombinantly produced apyrase. Preferably, the apyrase is recombinantly produced apyrase having the sequence of SEQ ID NO: 1 or a variant thereof, e.g. having at least 70% or 75%, more preferably at least 80% or 85%, even more preferably at least 90% or 95%, still more preferably at least 97% or 98%, such as at least 99% sequence identity, wherein R192 is preferably maintained, or recombinantly produced apyrase having the sequence of SEQ ID NO: 2 or a variant thereof, e.g. having at least 70% or 75%, more preferably at least 80% or 85%, even more preferably at least 90% or 95%, still more preferably at least 97% or 98%, such as at least 99% sequence identity; wherein R192 is preferably maintained. More preferably, the apyrase or a fragment thereof is an isolated and purifed recombinant apyrase or a fragment thereof, even more preferably a recombinant apyrase from *Shigella flexneri* or a fragment thereof e.g. an isolated and purifed recombinant apyrase from *Shigella flexneri* or a fragment thereof, or a recombinant apyrase from Solanum tuberosum or a fragment thereof e.g. an isolated and purifed recombinant apyrase from Solanum tuberosum or a fragment thereof, in particular a recombinant apyrase from *Shigella flexneri* or a fragment thereof, more particular an isolated and purifed recombinant apyrase from *Shigella flexneri* or a fragment thereof, even more particular a recombinant apyrase or a fragment thereof, e.g. an isolated and purifed recombinant apyrase from *Shigella flexneri* comprising the amino acid sequence as shown in SEQ ID NO: 1 or SEQ ID NO: 2, preferably the amino acid sequence as shown in SEQ ID NO: 1, or variants thereof as described above. For recombinant production, the ATP-hydrolyzing enzyme may be encoded by a nucleic acid not naturally occurring in the cell or organism expressing the ATP-hydrolyzing enzyme. Recombinant production of the ATP-hydrolyzing enzyme may be achieved, for example, (1) by heterologous expression (wherein the apyrase sequence is derived from a different organism than the organism used for its expression), (2) by expression based on an expression vector (not occurring in nature; e.g. for overexpression of the ATP-hydrolyzing enzyme), (3) by not naturally occurring ATP-hydrolyzing enzymes (e.g., functional variants as described above), or by any combination of (1) - (3). A (heterologous) cell expressing the ATP-hydrolyzing enzyme may impart a post-translational modification (PTM; e.g., glycosylation) on the ATP-hydrolyzing enzyme that is not present in its native state. Such a PTM may result in a functional difference (e.g., reduced immunogenicity). Accordingly, the ATP-hydrolyzing enzyme may have a post-translational modification, which is distinct from the naturally produced ATP-hydrolyzing enzyme. As an alternative, the apyrase may be used directly from a natural source. The apyrase may be obtained from a plant source, an animal source or a bacterial source. The apyrase may be purified or cell extracts (such as periplasmic extracts of bacterial cells) may be used.

The composition of the present invention is usually produced in a reaction vessel like a reactor. The formation of the protective layer is usually carried out by forming the respective protective layer by building blocks, wherein the building blocks build the protective layer in a polycondensation reaction. The polycondensation can be effected in different solvents, preferably in aqueous solution. Polycondensation can be easily controlled and stopped if appropriate, allowing achievement of a defined thickness of the protective layer. The choice of the building blocks, which can be used to build the protective layer, may depend on the known structure of the ATP-hydrolyzing enzyme in order to adapt the affinity of the protective layer according to optimal and/or desired parameters. As building blocks for the protective layer usually structural building blocks and protective building blocks are used to build the protective layer. Structural building blocks which can be used are e.g. tetraethylorthosilicate (designated herein as "TEOS" or "T"). Protective building blocks which can be used are e.g. 3-Aminopropyltriethoxysilane (designated herein as "APTES" or "A"), Propyltriethyoxysilane (designated herein as "PTES" or P"), Isobutyltriethoxysilane (designated as "IBTES"), Hydroxymethyltriethoxysilane (designated herein as "HTMEOS" or H), Benzyltriethoxysilane (designated herein as "BTES"), Ureidopropyltriethoxysilane (designated as "UPTES"), or Carboxyethyltriethoxysilane (designated herein as "CETES"). Structural building blocks are usually precursors of inorganic silica, capable of forming 4 covalent bonds in the layer formed. Protective building blocks are usually organosilanes, bearing an organic moiety endowed with the ability to interact with the ATP-hydrolyzing enzymes. Preferred structural building blocks are tetravalent silanes, in particular tetra-alkoxy-silanes. Preferred protective building blocks are trivalent silanes, in particular tri-alkoxy-silanes. More preferred structural building blocks are mixtures of tetravalent silanes and trivalent silanes, in particular mixtures of tetra-alkoxy-silanes and tri-alkoxy-silanes. Even more preferred structural building blocks are selected from the group consisting of tetraethylorthosilicate, tetra-(2-hydroxyethyl)silane, and tetramethylorthosilicate. Even more preferred protective building blocks are selected from the group consisting of carboxyethylsilanetriol, benzylsilanes, propylsilanes, isobutylsilanes, n-octylsilanes, hydroxysilanes, bis(2-hydroxyethyl)-3 -aminopropylsilanes, aminopropylsilanes, ureidopropylsilanes, (N-Acetylglycyl)-3-aminopropylsilanes, hydroxy(polyethyleneoxy)propyl]triethoxysilanes, in particular selected from benzyltriethoxysilane, propyltriethoxysilane, isobutyltriethoxysilane, n-octyltriethoxysilane, hydroxymethyltriethoxysilane, bis(2-hydroxyethyl)-3 -aminopropyltriethoxysilane, 3-Aminopropyltriethoxysilane, ureidopropyltriethoxysllane, (N-Acetylglycyl)-3-aminopropyltriethoxysilane, or selected from benzyltrimethoxysflane, propyltrimethoxysilane, isobutylimethoxysilane, n-octyltrimethoxysilane, hydroxymethyltrimethoxysilane, bis(2-hydroxyethyl)-3 -aminopropyltrimethoxysilane, arninopropyltrimethoxysilane, ureidopropyltrimethoxysilane (N-Acetylglycyl)-3 -aminopropyltrimethoxysilane or selected from benzyltrihydroxyethoxysilane, propyltrihydroxyethoxysilane, isobutyltrihydroxyethoxysilane, n-octyltrihydroxyethoxysilane, hydroxymefilyltrihydroxyethoxysilane, bis(2-hydroxyethyl)-3 - aminopropyltrihydroxyethoxysilane, aminopropyltrihydroxyethoxysilane, Ureidopropyltrihydroxyethoxysilane (N-Acetylglycyl)-3-aminopropyltrihydroxymethoxysilane.

Particular preferred building blocks are TEOS as structural building block and APTES, PTES, and/or HTMEOS, preferably APTES as protective building block. In particular TEOS as structural building block and APTES as protective building block are used to build the protective layer.

The reaction time of the building blocks with the solid carrier depends on the length of the linker, if a linker is used, and the size of the ATP-hydrolyzing enzyme. The reaction is usually carried out for a time period of between 0.5 to 10 hours, preferably between 1 and 5 hours, more preferably between 1 and 4 hours, even more preferably between 2 and 4 hours, preferably in aqueous solution and preferably at room temperature of about 5 to about 25 °C or at about 20 °C. The formation of the protective layer can be stopped by actively stopping the polycondensation reaction e.g by removing the non-reacted building blocks e.g. by a washing step or by self-stopping of the polycondensation reaction caused by a limited amount of buidling blocks.

In a furthermore preferred embodiment the ATP-hydrolyzing enzyme is immobilized on the solid carrier by at least partly modifying the surface of the solid carrier by introducing a molecule as anchoring point as described supra for the ATP-hydrolyzing enzyme and by using a linker, preferably a cross-linker binding to the anchoring point and the ATP-hydrolyzing enzyme.

In one embodiment the introduced molecule as anchoring point and/or the linker are homogeneously distributed on the surface of the solid carrier.

In a preferred embodiment the cross-linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils, sulfhydryl-reactive 2-pyridyldithiol, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), Sulfo-LC-SMPT (4-Sulfosuccinimidyl-6-methyl-a-(2-pyridyldithio)toluamido]hexanoate)), SPDP (N-Succinimidyl 3-(2-pyridyldithio)-propionate), LC-SPDP (Succinimidyl 6-(3-[2-pyridyldithio]-propionamido)hexanoate), SMPT (4-Succinimidyloxycarbonyl-methyl-a-[2-pyridyldithio]toluene), DPDPB (1,4-Di-[3'-(2'-pyridyldithio)-propionamido]butane), DTME (Dithio-bismaleimidoethane), BMDB (1,4 bismaleimidyl-2,3-dihydroxybutane). More preferably said cross-linker is selected from glutaraldehyde, disuccinimidyl tartrate, disuccinimidyl suberate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils (e.g. suflhydryl-reactive 2-pyridyldithio). In a more preferred embodiment the cross-linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate·2 HCl), DST (Disuccinimidyl tartarate), BMDB (1,4 bismaleimidyl-2,3-dihydroxybutane).

More preferably said cross-linker is selected from glutaraldehyde, disuccinimidyl tartrate, disuccinimidyl suberate, bis[sulfosuccinimidyl] suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils (e.g. suflhydryl-reactive 2-pyridyldithio). Most preferred is glutaraldehyde.

After the protective layer has been formed, the solid carrier comprising the ATP-hydrolyzing enzyme and the protective layer can be stored. Storing is usually accomplished e.g. by washing the composition formed e.g. with a buffer and storing it suspended or solved in that buffer for a desired time period. In a preferred embodiment the solid carrier comprising the ATP-hydrolyzing enzyme and the protective layer is stored at a constant temperature between 2 to 25 °C. In a further preferred embodiment, the solid carrier comprising the ATP-hydrolyzing enzyme and the protective layer is stored 5 to 48 hours, preferably 10 to 30 hours. More preferably the solid carrier comprising the ATP-hydrolyzing enzyme and the protective layer is stored at a constant temperature between 2 to 25 °C, preferably at room temperature for 10 to 30 hours.

In one embodiment, the functional constituent binds to mucus.

In one embodiment a polymer comprising repeat units wherein each repeat unit comprises at least one amino group and/or at least one thiol group is a polymer comprising repeat units wherein each repeat unit comprises at least one amino group.

In one embodiment a polymer comprising repeat units wherein each repeat unit comprises at least one amino group and/or at least one thiol group is a polymer comprising repeat units wherein each repeat unit comprises at least one thiol group.

In one embodiment a polymer comprising repeat units wherein each repeat unit comprises at least one amino group and/or at least one thiol group is selected from the group consisting of a polyglucosamin, a polymerized silane-PEG-NH2 and a polymerized silane comprising an amino group. In a preferred embodiment the polymer comprising repeat units wherein each repeat unit comprises at least one amino group and/or at least one thiol group, is selected from the group consisting of a polyglucosamin, a polymerized silane-PEG-NH2 and polymerized APTES.

In a more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; and a polymerized silane comprising an amino group, preferably a polymerized APTES. In an even more preferred embodiment the polymer comprising repeat units wherein each repeat unit comprises at least one amino group and/or at least one thiol group, is a polyglucosamin, preferably a polyglucosamin selected from the group consisting of chitin, chitosan, polyglucosaminoglycans, chondroitin, heparin, keratan and dermatan or a derivative thereof, more preferably a chitosan or a derivative thereof. The most preferred polymer comprising repeat units wherein each repeat unit comprises at least one amino group and/or at least one thiol group is a chitosan or a derivative thereof.

A preferred polyglucosamin of the present invention is selected from the group consisting of chitin, chitosan, polyglucosaminoglycans, chondroitin, heparin, keratan and dermatan or a derivative thereof. Most preferred is a chitosan or a derivative thereof. A preferred silane-PEG-NH2 of the polymerized silane-PEG-NH2 is selected from the group consisting of silane-PEG4-NH2, silane-PEG2000-NH2, and silane-PEG5000-NH2. A preferred polymerized silane comprising an amino group is selected from the group consisting of APTES, amino-butyl-TES, amino-pentyl-TES, amino-hexyl-TES, amino-heptyl-TES, and amino-octyl-TES, and is in particular APTES.

In a further embodiment a polymer comprising repeat units wherein each repeat unit comprises at least one amino group and/or at least one thiol group is selected from the group consisting of a polyglucosamin, a polymerized silane-PEG-NH2, a polymerized silane comprising an amino group, a polymerized silane comprising a thiol group, a polycarbophil-cysteine conjugate, a polymerized silane-PEG-thiol and a polycysteine. In a further more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; a polymerized silane comprising a thiol group, preferably a polymerized MPTS; a polycarbophil-cysteine conjugate; a polymerized silane-PEG-thiol; and a polycysteine. In an even more preferred embodiment the polymer comprising repeat units wherein each repeat unit comprises at least one amino group and/or at least one thiol group, is a polyglucosamin or a polymerized silane comprising a thiol group, preferably a polyglucosamin selected from the group consisting of chitin, chitosan, polyglucosaminoglycans, chondroitin, heparin, keratan and dermatan or a derivative thereof, more preferably a chitosan or a derivative thereof or a polymerized silane comprising a thiol group, a polycarbophil-cysteine conjugate, and a polymerized silane-PEG-thiol, preferably a polymerized silane comprising a thiol group.

In a particular embodiment, the polymer comprising repeat units wherein each repeat unit comprises at least one amino group and/or at least one thiol group, is selected from the group consisting of chitin, chitosan, polyglucosaminoglycans, chondroitin, heparin, keratin, dermatan or a derivative thereof in particular chitosan or a derivative thereof, a polymerized silane-PEG-NH2 selected from the group consisting of polymerized silane-PEG4-NH2, polymerized silane-PEG2000-NH2, polymerized silane-PEG5000-NH2, a polymerized silane comprising an amino group which is preferably polymerized APTES and a polymerized silane comprising a thiol group, which is preferably polymerized MPTS.

In one embodiment a polymer comprising repeat units wherein each repeat unit comprises at least one amino group and/or at least one thiol group is selected from the group consisting of a polyglucosamin, a polymerized silane-PEG-NH2, a polymerized silane comprising an amino group and a polymerized silane comprising a thiol group. In a preferred embodiment the polymer comprising repeat units wherein each repeat unit comprises at least one amino group and/or at least one thiol group, is selected from the group consisting of a polyglucosamin, a polymerized silane-PEG-NH2, polymerized APTES and polymerized MPTS.

In a more preferred embodiment the polymer comprising repeat units wherein each repeat unit comprises at least one amino group and/or at least one thiol group is selected from the group consisting of a polyglucosamin selected from the group consisting of chitin, chitosan, polyglucosaminoglycans, chondroitin, heparin, keratan and dermatan or a derivative thereof; a polymerized silane-PEG-NH2; a polymerized silane comprising an amino group, preferably a polymerized APTES; and a polymerized silane comprising a thiol group, preferably polymerized MPTS. In a particular embodiment, the polymer comprising repeat units wherein each repeat unit comprises at least one amino group and/or at least one thiol group, is selected from the group consisting of chitin, chitosan, polyglucosaminoglycans, chondroitin, heparin, keratin, dermatan or a derivative thereof, most particular chitosan or a derivative thereof.

In one embodiment a polymer comprising repeat units wherein each repeat unit comprises at least one thiol group is selected from the group consisting of a polymerized silane comprising a thiol group, a polycarbophil-cysteine conjugate, a polymerized silane-PEG-thiol and a polycysteine, and is preferably selected from the group consisting of a polymerized silane comprising a thiol group, a polycarbophil-cysteine conjugate, and a polymerized silane-PEG-thiol, and is more preferably a polymerized silane comprising a thiol group, and is most perferably polymerized MPTS. In one embodiment a polymerized silane comprising a thiol group is preferably polymerized MPTS.

In one embodiment 5% to 100%, preferably 10% to 100%, more preferably 50% to 100%, of the surface of the protective layer is covered with a polymer comprising repeat units wherein each repeat unit comprises at least one amino group and/or at least one thiol group.

In one embodiment the functional constituent is immobilized on the surface of the protective layer by binding, preferably covalent binding. In a preferred embodiment the functional constituent is immobilized on the surface of the protective layer by non-covalent binding, preferably by electrostatic interactions. In a more preferred embodiment the polymer comprising repeat units wherein each repeat unit comprises at least one amino group and/or at least one thiol group is immobilized on the surface of the protective layer by covalent binding.

In one embodiment the functional constituent is immobilized on the surface of the protective layer using a spacer binding to the surface of the protective layer and the functional constituent. Thus in one embodiment the present invention comprises a composition comprising a solid carrier, an ATP-hydrolyzing enzyme or a fragment thereof immobilized on the surface of the solid carrier, a protective layer to protect the ATP-hydrolyzing enzyme or a fragment thereof by embedding the ATP-hydrolyzing enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group, wherein the functional constituent is immobilized on the surface of the protective layer by a spacer. Examples of such a spacer include a polyethylene such as PEG4, PEG2000, PEG5000. A functional constituent immobilized on the surface of the protective layer, by a spacer is usually produced by firstly reacting the spacer with the functional constituent, so that the spacer binds to the functional constituent and then the functional constituent bound to the spacer is reacted with the the surface of the protective layer.

The immobilization of the functional constituent to the surface of the protective layer is usually carried out in a reaction vessel like a reactor by suspending the solid carrier carrying the ATP-hydrolyzing enzyme embedded in a protective layer as described supra in e.g. in water, buffer or non-ionic surfactants or mixtures thereof, preferably in mixtures of water and non-ionic surfactants. The functional component is then added to the suspension to react usually under stirring with the surface of the protetctive layer to immobilize the functional constitutent on the surface of the protective layer. Ususally such obtained composition is washed and resuspended into water, buffer or non-ionic surfactants or mixtures thereof. Immobilization takes place by non-covalent binding e.g. electrostatic binding or by covalent binding of the functional constituent. The functional constituent may be immobilized by chemically modifying the surface of the protective layer and the functional constituent using e.g. "click chemistry" such as copper-catalyzed click chemistry (Copper-catalysed azide-alkyne cycloaddition, see e.g. Kolb et al. (2001) Angew. Chem. 40(11)2004-2021) or by copper free click chemistry (Wittig G, A Chem Ber, 1961, 94, 3260) ., e.g. the solid carrier carrying the ATP-hydrolyzing enzyme embedded in a protetctive layer as described supra is first reacted with a reactive compound like an ethynyl compound and the functional constituent is modified by adding a reactive compound e.g.an azide residue and then both components are reacted to immobilize the functional constituent on the surface of the protective layer.

A preferred composition comprises a solid carrier, whereby the solid carrier is a silica nanoparticle (SNP), an ATP-hydrolyzing enzyme or a fragment thereof immobilized on the surface of the solid carrier, wherein the ATP-hydrolyzing enzyme or a fragment thereof is a apyrase or a fragment thereof, a protective layer to protect the ATP-hydrolyzing enzyme or a fragment thereof by embedding the ATP-hydrolyzing enzyme or a fragment thereof, wherein the protective layer comprises tetravalent silanes and trivalent silanes; and a functional constituent immobilized on the surface of the protective layer, wherein the functional constituent immobilized on the surface of the protective layer is a polymer comprising repeat units wherein each repeat unit comprises at least one amino group, preferably wherein the functional constituent immobilized on the surface of the protective layer is chitosan.

In a further aspect the present invention provides the composition as described supra for use as a medicament.

In a further aspect the present invention provides the composition for use in a method for the prevention, delay of progression or treatment of dysbiosis or a dysbiosis-related disease. Also provided is the use of the composition as described herein for the manufacture of a medicament for the prevention, delay of progression or treatment of dysbiosis or a dysbiosis-related disease in a subject. Also provided is the use of the composition as described herein for the prevention, delay of progression or treatment of dysbiosis or a dysbiosis-related disease in a subject. Also provided is a method for the prevention, delay of progression or treatment of dysbiosis or a dysbiosis-related disease in a subject, comprising administering to said subject a therapeutically effective amount of the composition as described herein.

Dysbiosis or a dysbiosis-related disease can be caused by dysbiosis-inducing agents. Dysbiosis-inducing agents are described, for example, in Le Bastard Q, Al-Ghalith GA, Grégoire M, et al. Systematic review: human gut dysbiosis induced by non-antibiotic prescription medications. Aliment Pharmacol Ther. 2018;47(3):332-345. doi:10.1111/apt.14451. Dysbiosis-inducing agents include dysbiosis-inducing antibiotics, chemotherapeutic agents, proton pump inhibitors, statins, immunosuppressive drugs (e.g., glucocorticoids), metformin, antipsychotics (e.g., atypical antipsychotics) and agents for oral iron supplementation.

In some embodiments, the dysbiosis-inducing agent may be an antibiotic. In other embodiments, the dysbiosis-inducing agent may be a non-antibiotic medication. Examples of dysbiosis-inducing non-antibiotic medications include chemotherapeutic agents, proton pump inhibitors, statins, immunosuppressive drugs (e.g., glucocorticoids), metformin, and antipsychotics (e.g., atypical antipsychotics). Preferably, the dysbiosis-inducing non-antibiotic medication is a chemotherapeutic agent or a proton pump inhibitor. In particular, the dysbiosis-inducing chemotherapeutic agent may be a cytotoxic or cytostatic agent. In some embodiments, the dysbiosis-inducing chemotherapeutic agent may be selected from the group consisting of alkylating agents, anthracyclines, cytoskeletal disruptors, epothilones, histone deacetylase inhibitors, inhibitors of topoisomerase I or II, kinase inhibitors, nucleotide analogs and precursor analogs, platinum-based agents, retinoids, and vinca alkaloids and derivatives. Specific, non-limiting examples of dysbiosis-inducing chemotherapeutic agents include 5-fluorouracil (5-FU) and irinotecan.

The dysbiosis-inducing antibiotic may be selected from the group consisting of penicillins, tetracyclines, cephalosporins, quinolones, lincosamides, macrolides, sulfonamides, glycopeptides, aminoglycosides, carbapenems, ansamycins, carbacephems, lipopeptides, monobactams, nitrofurans, oxazolidinones, and polypeptides.

In some embodiments, the antibiotic may be an aminoglycoside. Non-limiting examples of aminoglycosides include amikacin, gentamicin, kanamycin, neomycin, netilmicin, tobramycin, paromomycin, streptomycin, and spectinomycin.

In some embodiments, the antibiotic may be an ansamycin. Non-limiting examples of ansamycins include geldanamycin, herbimycin, and rifaximin.

In some embodiments, the antibiotic may be a carbacephem, such as loracarbef.

In some embodiments, the antibiotic may be a carbapenem. Non-limiting examples of carbapenems include ertapenem, doripenem, imipenem/cilastatin, and meropenem.

In some embodiments, the antibiotic may be a cephalosporin (e.g., first, second, third, fourth or fifth generation). Non-limiting examples of first-generation cephalosporins include cefadroxil, cefazolin, cephradine, cephapirin, cephalothin, and cefalexin. Non-limiting examples of second-generation cephalosporins include cefaclor, cefoxitin, cefotetan, cefamandole, cefmetazole, cefonicid, loracarbef, cefprozil, and cefuroxime. Non-limiting examples of third-generation cephalosporins include cefixime, cefdinir, cefditoren, cefoperazone, cefotaxime, cefpodoxime, ceftazidime, ceftibuten, ceftizoxime, moxalactam, and ceftriaxone. Non-limiting examples of fourth-generation cephalosporins include cefepime. Non-limiting examples of fifth-generation cephalosporins include ceftaroline, fosamil and ceftobiprole.

In some embodiments, the antibiotic may be a glycopeptide antibiotic. Non-limiting examples of glycopeptide antibiotics include teicoplanin, vancomycin, telavancin, dalbavancin, and oritavancin.

In some embodiments, the antibiotic may be a lincosamide. Non-limiting examples of lincosamides include clindamycin and lincomycin.

In some embodiments, the antibiotic may be a lipopeptide antibiotic, such as daptomycin.

In some embodiments, the antibiotic may be a macrolide. Non-limiting examples of macrolides include azithromycin, clarithromycin, erythromycin, roxithromycin, telithromycin, spiramycin, and fidaxomicin.

In some embodiments, the antibiotic may be a monobactam, such as aztreonam.

In some embodiments, the antibiotic may be a nitrofuran. Non-limiting examples of nitrofurans include furazolidone and nitrofurantoin.

In some embodiments, the antibiotic may be an oxazolidinone. Non-limiting examples of oxazolidinones include linezolid, posizolid, radezolid and torezolid.

In some embodiments, the antibiotic may be a penicillin. Non-limiting examples of penicillins include amoxicillin, ampicillin, azlocillin, dicloxacillin, flucloxacillin, mezlocillin, methicillin, nafcillin, oxacillin, penicillin G, penicillin V, piperacillin, penicillin G, temocillin, and ticarcillin.

In some embodiments, the antibiotic may be a polypeptide antibiotic. Non-limiting examples of polypeptide antibiotics include bacitracin, colistin and polymyxin B.

In some embodiments, the antibiotic may be a quinolone/fluoroquinolone. Non-limiting examples of quinolones/fluoroquinolones include ciprofloxacin, enoxacin, gatifloxacin, gemifloxacin, levofloxacin, lomefloxacin, moxifloxacin, nadifloxacin, nalidixic acid, norfloxacin, ofloxacin, trovafloxacin, grepafloxacin, sparfloxacin, and temafloxacin.

In some embodiments, the antibiotic may be a sulfonamide. Non-limiting examples of sulfonamides include mafenide, sulfacetamide, sulfadiazine, silver sulfadiazine, sulfadimethoxine, sulfamethizole, sulfamethoxazole, sulfanilimide, sulfasalazine, sulfisoxazole, trimethoprim-sulfamethoxazole, and sulfonamidochrysoidine.

In some embodiments, the antibiotic may be a tetracyclin. Non-limiting examples of tetracyclins include demeclocycline, doxycycline, metacycline, minocycline, oxytetracycline, and tetracycline.

Accordingly, non-limiting examples of antibiotics include amikacin, gentamicin, kanamycin, neomycin, netilmicin, tobramycin, paromomycin, streptomycin, spectinomycin, geldanamycin, herbimycin, rifaximin, loracarbef, ertapenem, doripenem, imipenem/cilastatin, meropenem, cefadroxil, cefazolin, cephradine, cephapirin, cephalothin, cefalexin, cefaclor, cefoxitin, cefotetan, cefamandole, cefmetazole, cefonicid, loracarbef, cefprozil, cefuroxime, cefixime, cefdinir, cefditoren, cefoperazone, cefotaxime, cefpodoxime, ceftazidime, ceftibuten, ceftizoxime, moxalactam, ceftriaxone, cefepime, ceftaroline fosamil, ceftobiprole, teicoplanin, vancomycin, telavancin, dalbavancin, oritavancin, clindamycin, lincomycin, daptomycin, azithromycin, clarithromycin, erythromycin, roxithromycin, telithromycin, spiramycin, fidaxomicin, aztreonam, furazolidone, nitrofurantoin, linezolid, posizolid, radezolid, torezolid, amoxicillin, ampicillin, azlocillin, dicloxacillin, flucloxacillin, mezlocillin, methicillin, nafcillin, oxacillin, penicillin G, penicillin V, piperacillin, penicillin G, temocillin, ticarcillin, bacitracin, colistin, polymyxin B, ciprofloxacin, enoxacin, gatifloxacin, gemifloxacin, levofloxacin, lomefloxacin, moxifloxacin, nadifloxacin, nalidixic acid, norfloxacin, ofloxacin, trovafloxacin, grepafloxacin, sparfloxacin, temafloxacin, mafenide, sulfacetamide, sulfadiazine, silver sulfadiazine, sulfadimethoxine, sulfamethizole, sulfamethoxazole, sulfanilimide, sulfasalazine, sulfisoxazole, trimethoprim-sulfamethoxazole, sulfonamidochrysoidine, demeclocycline, doxycycline, metacycline, minocycline, oxytetracycline, tetracycline, arsphenamine, chloramphenicol, fosfomycin, fusidic acid, metronidazole, mupirocin, platensimycin, quinupristin/dalfopristin, thiamphenicol, tigecycline, tinidazole, and trimethoprim.

In some embodiments, the antibiotic may be a penicillin, such as ampicillin. In some embodiments, the antibiotic may be a (third generation) cephalosporin, such as cefoperazone. In some instances, the antibiotic may be a glycopeptide-antibiotic, such as vancomycin. In certain embodiments, the antibiotic may be metronidazole. Particularly preferably, the antibiotic may be selected from the group consisting of vancomycin, ampicillin, metronidazole and cefoperazone; in particular the antibiotic may be ampicillin or cefoperazone.

In one embodiment dysbiosis is induced by an antibiotic agent e.g. an antibiotic agent as described above, a chemotherapeutic agent e.g. a chemotherapeutic agent as described above, a diet or by maternal dysbiosis.

In one embodiment the dysbiosis-related disease is selected from inflammatory diseases, gastrointestinal tract-related disorders, metabolic disorders, CNS-related disorders, cancers and autoimmune diseases.

In one embodiment the dysbiosis-related disease is selected from inflammatory bowel disease, irritable bowel syndrome, obesity, diabetes, metabolic syndrome, coeliac disease, colorectal cancer, Clostridioides difficile infection, autism spectrum disorder, urinary stone disease (USD), lupus erythematosus, rheumatoid arthritis, systemic sclerosis, Sjogren's syndrome, anti-phospholipid syndrome, cardiovascular syndrome, allergy, and asthma.

In one embodiment the composition is administered in combination with a dysbiosis-inducing agent. Preferably the dysbiosis- inducing agent is an antibiotic; preferably selected from the group consisting of penicllins, tetracyclines, cephalosporins, quinolones, lincosamides, macrolides, sulfonamides, glycopeptides, aminoglycosides, carbapenems, ansamycins, carbacephems, lipopeptides, monobactams, nitrofurans, oxazolidinones, and polypeptides; more preferably the antibiotic is selected from the group consisting of vancomycin, ampicillin, metronidazole and cefoperazone. Equally preferably the dysbiosis-inducing agent is a chemotherapeutic agent; preferably selected from alkylating agents, anthracyclines, cytoskeletal disrupters, epothilones, histone deacetylase inhibitors, inhibitors of topoisomerase I or II, kinase inhibitors, nucleotide analogs and precursor analogs, platinum-based agents, retinoids, and vinca alkaloids and derivatives; for example the chemotherapeutic agent is 5-fluorouracil (5-FU).

In one embodiment dysbiosis or a dysbiosis-related disease is caused by malnutrition, neonatal sepsis or unhealthy diet.

In one embodiment dysbiosis or a dysbiosis-related disease is an inflammatory disease, preferably an inflammatory disease selected from the group consisting of pancreatitis, gingivitis, periodontitis, inflammatory bowel disease (IBD), Crohn's disease (CD), ulcerative colitis (UC), gastritis, enteritis, esophagitis, diverticulitis, rheumatoid arthritis and infectious colitis (IBD).

In a preferred embodiment dysbiosis or a dysbiosis-related disease is selected from the group consisting of ulcerative colitis , Crohn's disease and Graft-versus-host disease.

In a more preferred embodiment dysbiosis or a dysbiosis-related disease is selected from the group consisting of ulcerative colitis (UC) and Crohn's disease, and is preferably ulcerative colitis (UC).

A composition according to the invention is preferably a pharmaceutical composition and comprises a therapeutically effective amount of the composition as described herein and one or more suitable pharmaceutically acceptable carrier. A pharmaceutical composition according to the invention is suitable for oral administration to a subject. If not indicated otherwise, a pharmaceutical composition according to the invention is prepared in a manner known per se.

An exemplary treatment regime entails administration once daily, twice daily, three times daily, every second day, twice per week, once per week. The composition, e.g. the pharmaceutical composition of the invention is usually administered on multiple occasions. Intervals between single dosages can be, for example, less than a day, daily, every second day, twice per week, or weekly. The composition, e.g. the pharmaceutical composition of the invention may be given as a continous uninterrupted treatment. The composition, e.g. the pharmaceutical composition of the invention may also be given in a regime in which the subject receives cycles of treatment interrupted by a drug holiday or period of non-treatment. Thus, the composition, e.g. the pharmaceutical composition of the invention may be administered according to the selected intervals above for a continuous period of one week or a part thereof, for two weeks, for three weeks for four weeks, for five weeks or for six weeks and then stopped for a period of one week, or a part thereof, for two weeks, for three weeks, for four weeks, for five weeks, or for six weeks.

The composition, e.g. the pharmaceutical composition of the present invention may conveniently be administered in unit dosage forms. Units ("U") of enzyme activity can be described in terms of weight or mass of substrate hydrolyzed per unit time. Units ("U") can be described in terms of umol substrate converted per minute (or umol/min).

The expression "effective amount" or "therapeutically effective amount" as used herein refers to an amount capable of invoking one or more of the desired effects in a subject receiving the composition of the present invention. Determination of a therapeutically effective amount is well within the capability of those skilled in the art, especially in light of the detailed disclosure provided herein.

The terms "treatment"/"treating" as used herein includes: (1) delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

As used herein, "delay of progression" means increasing the time to appearance of a symptom of e.g. treatment of dysbiosis or a dysbiosis-related disease or a mark associated with e.g. treatment of dysbiosis or a dysbiosis-related disease or slowing the increase in severity of a symptom of dysbiosis or a dysbiosis-related disease. Further, "delay of progression" as used herein includes reversing or inhibition of disease progression. "Inhibition" of disease progression or disease complication in a subject means preventing or reducing the disease progression and/or disease complication in the subject.

Preventive treatments comprise prophylactic treatments. In preventive applications, the pharmaceutical combination of the invention is administered to a subject suspected of having, or at risk for developing the above mentioned diseases or disorders e.g. treatment of dysbiosis or a dysbiosis-related disease. In therapeutic applications, the pharmaceutical combination is administered to a subject such as a patient already suffering from the above mentioned diseases or disorders e.g. treatment of dysbiosis or a dysbiosis-related disease, in an amount sufficient to cure or at least partially arrest the symptoms of the disease. Amounts effective for this use will depend on the severity and course of the disease, previous therapy, the subject's health status and response to the drugs, and the judgment of the treating physician.

In the case wherein the subject's condition does not improve, the pharmaceutical combination of the invention may be administered chronically, which is, for an extended period of time, including throughout the duration of the subject's life in order to ameliorate or otherwise control or limit the symptoms of the subject's disease or condition.

In the case wherein the subject's status does improve, the pharmaceutical combination may be administered continuously; alternatively, the dose of drugs being administered may be temporarily reduced or temporarily suspended for a certain length of time (i.e., a "drug holiday"). Once improvement of the patient's condition has occurred, a maintenance dose of the pharmaceutical combination of the invention is administered if necessary. Subsequently, the dosage or the frequency of administration, or both, is optionally reduced, as a function of the symptoms, to a level at which the improved disease is retained.

The "treatment" of dysbiosis or a dysbiosis-related disease may be a prophylactic treatment (e.g., reducing the risk of occurrence) or a therapeutic treatment. As used herein, the term "therapeutic treatment" refers to treatment after the onset of dysbiosis or a dysbiosis-related disease, while "prophylactic treatment" refers to treatment before the onset of dysbiosis or a dysbiosis-related disease or before the first symptoms occur. In particular, "therapeutic treatment" does not include prophylactic measures applied before the onset of dysbiosis or a dysbiosis-related disease. Since the onset of dysbiosis or a dysbiosis-related disease is often associated with symptom(s) of dysbiosis or the dysbiosis-related disease, human or animal subjects are often "therapeutically" treated after the diagnosis or at least a (strong) assumption that the subject suffers from certain dysbiosis or a dysbiosis-related disease. Therapeutic treatment aims in particular at (1) ameliorating, reducing, improving, or curing a disease (state) or (2) at inhibiting or delaying the progression of a disease. However, prevention of the onset of a disease cannot typically be achieved by therapeutic treatment. Prophylactic treatment includes reducing the risk of occurrence of dysbiosis or a dysbiosis-related disease or reducing the degree of dysbiosis or a dysbiosis-related disease (when it occurs) in a prophylactic manner. For example, (prophylactic or therapeutic) treatment of dysbiosis may be considered as prophylactic treatment of diseases induced by dysbiosis.

In a further aspect the present invention provides a pharmaceutical combination comprising
(i) the composition as decribed herein above; and
(ii) an immune checkpoint modulator.

In one embodiment the immune checkpoint modulator is an inhibitor of an inhibitory checkpoint molecule (checkpoint inhibitor).

In one embodiment the inhibitory checkpoint modulator is selected from A2AR, B7-H3, B7-H4, BTLA, CD40, CTLA-4, IDO, KIR, LAG3, PD-1, PDL-1, PD-L2, TIM-3, VISTA, CEACAM1, GARP, PS, CSF1R, CD94/NKG2A, TDO, TNFR, TIGIT and FasR/DcR3.

In one embodiment the immune checkpoint modulator is an inhibitor of A2AR, B7-H3, B7-H4, BTLA, CD40, CTLA-4, IDO, KIR, LAG3, PD-1, TIM-3, VISTA, CEACAM1, GARP, PS, CSF1R, CD94/NKG2A, TDO, TNFR, TIGIT or DcR3; or an inhibitor of a ligand thereof. In one embodiment the immune checkpoint modulator is an inhibitor of the CTLA-4 pathway or the PD-1 pathway.

In one embodiment the immune checkpoint modulator is PD-1 or PD-L1.

In a further aspect the present invention provides a pharmaceutical combination comprising
(i) the composition as described herein above; and
(ii) an immune checkpoint modulator;
for use in a method for the prevention, delay of progression or treatment of cancer and/or for use in adoptive (T) cell therapy.

Also provided is the use of the pharmaceutical combination as described herein for the manufacture of a medicament for the prevention, delay of progression or treatment of cancer and/or in adoptive (T) cell therapy in a subject. Also provided is the use of the pharmaceutical combination as described herein for the prevention, delay of progression or treatment of cancer and/or in adoptive (T) cell therapy in a subject. Also provided is a method for the prevention, delay of progression or treatment of cancer and/or in adoptive (T) cell therapy in a subject, comprising administering to said subject a therapeutically effective amount of the c pharmaceutical combination as described herein. The immune checkpoint modulator for use is as described above.

In general, a "combination" of (i) composition as described herein above and of (ii) an immune checkpoint modulator, means that both components can exert their effects in a combined manner. To this end, the time window of the effects of both components usually overlaps. Accordingly, the effects of both components are usually present in the human or animal body at the same time (even if one or both of the components may be no longer physically present). In some embodiments, both components may be (physically) present in the human or animal body at the same time. Accordingly, the treatment with the immune checkpoint modulator preferably overlaps with the treatment with the composition as described herein above. Even if one component (i) or (ii) may not be administered, e.g., at the same day, as the other component (the other of (i) or (ii)), their treatment schedules are usually intertwined.

In a further aspect the present invention provides a method of producing a composition as described supra, e.g. a composition comprising a solid carrier, a ATP-hydrolyzing enzyme or a fragment thereof immobilized on the surface of the solid carrier, a protective layer to protect the ATP-hydrolyzing enzyme or a fragment thereof by embedding the ATP-hydrolyzing enzyme or a fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group; the method comprising the following steps:
(a) providing a solid carrier;
(b) immobilizing an ATP-hydrolyzing enzyme or a fragment thereof on the solid carrier;
(c) forming a protective layer on the surface of the solid carrier to protect the ATP-hydrolyzing enzyme or the fragment thereof immobilized on the solid carrier;
(d) immobilizing a functional constituent on the surface of the protective layer, wherein the functional constituent immobilized on the surface of the protective layer is a polymer comprising repeat units wherein each repeat unit comprises at least one amino group and/or at least one thiol group.

Step (a) is usually carried out by providing the solid carrier in suspension in water, non-ionic surfactants or a buffer or mixtures thereof, preferably in suspension in water and/or non-ionic surfactants, more preferably in suspension in water and/or non-ionic surfactants wherein no buffer is present in the suspension, even more preferably in suspension in mixtures of water and non-ionic surfactants in particular in suspension in mixtures of water and non-ionic surfactants wherein no buffer is present in the suspension. The suspension can be stirred e.g at 400 rpm, 20°C for 30 min. The immobilization of the ATP-hydrolyzing enzyme on the solid carrier in step b) of the present method is usually carried out by adding a solution of the ATP-hydrolyzing enzyme to the suspension of the solid carrier. Preferably a linker to connect the solid carrier with the ATP-hydrolyzing enzyme is added to the suspension of the solid carrier prior to adding the solution of the ATP-hydrolyzing enzyme to the suspension of the solid carrier. In a preferred embodiment the immobilization of the ATP-hydrolyzing enzyme on the solid carrier is carried out by providing a suspension of the solid carrier and adding a solution of the ATP-hydrolyzing enzyme, wherein the suspension with the added solution of the ATP-hydrolyzing enzyme is incubated to allow the enzyme to bind on the surface of the solid carrier. In a more preferred embodiment the immobilization of the ATP-hydrolyzing enzyme or a fragment thereof on the solid carrier in step b) is carried out by i) adding a linker to the solid carrier provided in step (a), preferably adding a linker to a suspension of the solid carrier provided in step a), and ii) adding the ATP-hydrolyzing enzyme or a fragment thereof, preferably adding a solution of the ATP-hydrolyzing enzyme or a fragment thereof, to the solid carrier and the linker or to the suspension comprising the solid carrier and the linker, wherein the linker connects the solid carrier with the ATP-hydrolyzing enzyme or a fragment thereof. In one embodiment, a building block of the protective layer, preferably a monomer of a building block of the protective layer, more preferably an organosilane, even more preferably a triethoxysilane, in particular APTES, is added to the solid carrier and the linker or to the suspension comprising the solid carrier and the linker, prior to adding the solution of the ATP-hydrolyzing enzyme or a fragment thereof. In a preferred embodiment the surface of the solid carrier is at least partly modified to improve immobilization of the ATP-hydrolyzing enzyme on the solid carrier. In particular, the surface of the solid carrier is at least partly modified before the ATP-hydrolyzing enzyme is immobilized. The surface of the solid carrier can be at least partly modified by adding a molecule as anchoring point for the ATP-hydrolyzing enzyme to the surface of the solid carrier as described supra.

The suspension comprising the solid carrier is usually incubated after each addition step described above to allow a reaction between e.g. the solid carrier and the molecule as anchoring point, the solid carrier and the linker and, the solid carrier comprising the linker and the ATP-hydrolyzing enzyme or a fragment thereof, respectively, so that the ATP-hydrolyzing enzyme or a fragment thereof connects the solid carrier, preferably the surface of the solid carrier, with the ATP-hydrolyzing enzyme or a fragment thereof via the linker, preferably by covalent binding, thereby immobilizing the ATP-hydrolyzing enzyme or a fragment thereof on the solid carrier.

In one embodiment in step (b) the ATP-hydrolyzing enzyme or a fragment thereof is immobilized on the solid carrier by connecting the solid carrier with the ATP-hydrolyzing enzymeor a fragment thereof via a linker, preferably by connecting the solid carrier with the ATP-hydrolyzing enzyme or a fragment thereof via a linker, wherein the solid carrier is connected with the ATP-hydrolyzing enzyme or a fragment thereof by covalent binding between the linker and the solid carrier and between the linker and the ATP-hydrolyzing enzyme or a fragment thereof. Preferably in step b), i) a linker is added to the solid carrier provided in step (a), and ii) the ATP-hydrolyzing enzyme or a fragment thereof is added to the solid carrier and the linker, wherein the linker connects the solid carrier with the ATP-hydrolyzing enzyme or a fragment thereof. The linker used is as described supra and connects the surface of the solid carrier with the ATP-hydrolyzing enzyme by preferably covalent binding. More preferably the linker is added to the solid carrier in step (b), in a molar excess to the ATP-hydrolyzing enzyme or a fragment thereof, preferably the linker is added to the solid carrier in step (b), in a 1 fold to 1000 fold molar excess to the ATP-hydrolyzing enzyme or a fragment thereof, more preferably the linker is added to the solid carrier in step (b), in a 2 fold to 300 fold molar excess to the ATP-hydrolyzing enzyme or a fragment thereof, even more preferably the linker is added to the solid carrier in step (b), in a 4 fold to 250 fold molar excess to the ATP-hydrolyzing enzyme or a fragment thereof, in particular the linker is added to the solid carrier in step (b), in a 25 fold molar excess to the ATP-hydrolyzing enzyme or a fragment thereof.

In a preferred embodiment the linker which has not connected the solid carrier with the ATP-hydrolyzing enzyme or a fragment thereof in step (b), is present during formation of a protective layer on the surface of the solid carrier in step (c). In a more preferred embodiment the linker which has not connected the solid carrier with the ATP-hydrolyzing enzyme or a fragment thereof in step (b), or a part thereof, covalently binds the protective layer to the ATP-hydrolyzing enzyme or the fragment thereof in step (c). In a furthermore preferred embodiment the linker which has not connected the solid carrier with the ATP-hydrolyzing enzyme or a fragment thereof in step (b) is not removed in step (b) or step (c) or in between step (b) and (c). In a particular embodiment the linker which has not connected the solid carrier with the ATP-hydrolyzing enzyme or a fragment thereof in step (b) is not removed in step (b) or step (c) or in between step (b) and (c) and the linker which has not connected the solid carrier with the ATP-hydrolyzing enzyme or a fragment thereof in step (b), or a part thereof, covalently binds the protective layer to the ATP-hydrolyzing enzyme or the fragment thereof in step (c). The amount of the linker which has not connected the solid carrier with the ATP-hydrolyzing enzyme or a fragment thereof in step (b) is usually between 30% and 70%, preferably between 40% and 60 %, more preferably around 50% of the amount of linker added to the solid carrier in step (b). In one embodiment there is no washing step between adding the linker to the solid carrier provided in step (a) in (i) and adding the ATP-hydrolyzing enzyme or a fragment thereof to the solid carrier and the linker in ii). In one embodiment there is no washing step between any of steps (a) to (c). In one embodiment there is no washing step between adding the linker to the solid carrier provided in step (a) in (i) and adding the ATP-hydrolyzing enzyme or a fragment thereof to the solid carrier and the linker in ii) and there is no washing step between any of steps (a) to (c).

In one embodiment the linker is is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, activated sulfhydrils, sulfhydryl-reactive 2-pyridyldithiol, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), Sulfo-LC-SMPT (4-Sulfosuccinimidyl-6-methyl-a-(2-pyridyldithio)toluamido]hexanoate)), SPDP (N-Succinimidyl 3-(2-pyridyldithio)-propionate), LC-SPDP (Succinimidyl 6-(3-[2-pyridyldithio]-propionamido)hexanoate), SMPT (4-Succinimidyloxycarbonyl-methyl-a-[2-pyridyldithio]toluene), DPDPB (1,4-Di-[3'-(2'-pyridyldithio)-propionamido]butane), DTME (Dithio-bismaleimidoethane), BMDB (1,4 bismaleimidyl-2,3-dihydroxybutane) and is preferably glutaraldehyde.

In a preferred embodiment the linker is selected from the group consisting of glutaraldehyde, disuccinimidyl tartrate, bis[sulfosuccinimidyl]suberate, ethylene glycolbis(sulfosuccinimidylsuccinate), dimethyl adipimidate, dimethyl pimelimidate, sulfosuccinimidyl (4-iodoacetyl) aminobenzoate, 1,5-difluoro-2,4-dinitrobenzene, BSOCOES (Bis[2-(succinimidooxycarbonyloxy)ethyl]sulfone), DSP (Dithiobis[succinimidyl]propionate]), DTSSP (3,3 '-Dithiobis[sulfosuccinimidyl]propionate]), DTBP (Dimethyl 3,3 '-dithiobispropionimidate 2 HCl), DST (Disuccinimidyl tartarate), BMDB (1,4 bismaleimidyl-2,3-dihydroxybutane) and is preferably glutaraldehyde.

The formation of the protective layer according to step (c) of the present method is usually carried out by forming the respective protective layer with building blocks, wherein the building blocks build the protective layer in a polycondensation reaction as described supa. The immobilization of a functional constituent on the surface of the protective layer according to step (d) of the present method is usually carried out as described supra.

In one embodiment the protective layer is formed by building blocks, wherein as building blocks structural building blocks and protective building blocks are used to form the protective layer, wherein the structural building blocks are precursors of inorganic silica, capable of forming 4 covalent bonds in the layer formed and the protective building blocks are organosilanes as described supra.

In one embodiment the protective layer embeds from about 30% to about 100% of the ATP-hydrolyzing enzyme.

In one embodiment the solid carrier is selected from the group of organic particles, inorganic particles, organic-inorganic particles, self-assembled organic particles, silica particles, gold particles, magnetic particles and titanium particles and is preferably a silica particle, more preferably a silica nanoparticle (SNP).

A preferred method of the present invention is a method of producing a composition, the composition comprising a solid carrier, a ATP-hydrolyzing enzyme or a fragment thereof immobilized on the surface of the solid carrier, a protective layer to protect the ATP-hydrolyzing enzyme or the fragment thereof by embedding the ATP-hydrolyzing enzyme or the fragment thereof, and a functional constituent immobilized on the surface of the protective layer, wherein the functional constituent immobilized on the surface of the protective layer is a polymer comprising repeat units, wherein each repeat unit comprises at least one amino group and/or at least one thiol group, the method comprising the following steps:
(a) providing a solid carrier, wherein the solid carrier is provided in suspension, preferably wherein the solid carrier is provided in suspension in water and/or non-ionic surfactants, more preferably wherein the solid carrier is provided in suspension in mixtures of water and non-ionic surfactants;
(b) immobilizing a ATP-hydrolyzing enzyme or a fragment thereof on the solid carrier, wherein preferably the surface of the solid carrier is at least partly modified before the ATP-hydrolyzing enzyme or a fragment thereof is immobilized on the solid carrier, wherein i) a linker is added to the suspension of the solid carrier or i) a linker is added to the suspension of the solid carrier after the at least partly modification of the surface of the solid carrier and ii) the ATP-hydrolyzing enzyme or a fragment thereof, preferably a solution of the ATP-hydrolyzing enzyme or a fragment thereof is added to the suspension of the solid carrier and the linker, wherein the linker connects the solid carrier with the ATP-hydrolyzing enzyme or a fragment thereof;
(c) forming a protective layer on the surface of the solid carrier to protect the ATP-hydrolyzing enzyme or the fragment thereof immobilized on the solid carrier, wherein the linker which has not connected the solid carrier with the ATP-hydrolyzing enzyme or a fragment thereof in step (b), or a part thereof, covalently binds the protective layer to the ATP-hydrolyzing enzyme or the fragment thereof;
(d) immobilizing a functional constituent on the surface of the protective layer, wherein the functional constituent immobilized on the surface of the protective layer is a polymer comprising repeat units, wherein each repeat unit comprises at least one amino group and/or at least one thiol group.

Also provided is a composition comprising a solid carrier, a ATP-hydrolyzing enzyme or a fragment thereof immobilized on the surface of the solid carrier, a protective layer to protect the ATP-hydrolyzing enzyme or the fragment thereof by embedding the ATP-hydrolyzing enzyme or the fragment thereof, and a functional constituent immobilized on the surface of the protective layer, wherein the functional constituent immobilized on the surface of the protective layer is a polymer comprising repeat units, wherein each repeat unit comprises at least one amino group and/or at least one thiol group, wherein the composition is obtainable by the methods, in particular by the preferred method of the invention as described supra.

### Examples

### Material and Methods:

### Reagents:

- Tetraethyl orthosilicate 99% (TEOS), (3-aminopropyl)-triethoxysilane (APTES), ammonium hydroxide (ACS grade, 28-30%), ethanol (ACS grade, anhydrous), glutaraldehyde (grade I, 25% in water), polysorbate 80, acetic acid, methanol, formic acid, trichloroacetic acid, sodium citrate tribasic dihydrate, NaOH, Na₂CO₃, potassium sodium tartrate, CuSO₄, Folin, D(+)Trehalose dihydrate and polyvinylpyrrolidone (PVP10K) were purchased from Sigma-Aldrich.
- Chitosan 95/500 P was purchased from Heppe Medical Chitosan GmbH.
- ATPase assay kit (colorimetric) was purchased from Abcam (ab234055).

### Mice

C57BL/6 mice maintained under specific pathogen-free (SPF) conditions were used. All animal experiments were performed in accordance with the Swiss Federal Veterinary Office guidelines and authorized by the Cantonal Veterinary. Animals were supervised by a licensed veterinarian and proper steps were taken to ensure the welfare and minimize the suffering of all animals in the conducted studies. Groups of 5 mice were housed in individually ventilated, filter-topped cages of approximately 355 cm2, maintained at room temperature (20-22° C) with a 12 h light/dark cycle, and free access to water and standard sterilized chow. The cages were sealed and autoclaved before use, and used in a 'Sealsafe' rack (Techniplast) with a 0.2 µm aerosol barrier vent. All manipulation of the cages (e.g. to replace bedding) took place in a cage changing station (CCS, Techniplast), designed to maintain animals in a sterile airflow environment. All experimental procedures and manipulation of the cages occurred in a sterile laminar flow hood (Skan AG).

### Synthesis of silica nanoparticles (SNPs)

Silica nanoparticles (50 nm) have been synthetized following the original Stöber process as described in WO2015/014888 A1. Briefly, ethanol, distilled water (6 M) and ammonium hydroxide (0.13 M) were mixed and stirred at 400 rpm for 1 h. TEOS (0.28 M) was added, and the solution was stirred at 400rpm at 20°C for 22h. The solution was then centrifuged at 20000 g for 20 min and washed successively with ethanol and water. Particle size measurement was carried out on SEM micrographs acquired at a magnification of 150000x using the image analysis software Olympus stream motion.

### Production of SNP-apyrase-AT

To SNPs (10 mg/mL, 62 nm) in H₂O/PS80 (8 mg/L) was added APTES (3.4 mM). The reaction mixture was allowed to react for 10 min at 20°C, 400 rpm. Then, glutaraldehyde (3.4 mM) was added, and the reaction mixture was stirred for 10 min at 20°C, 400 rpm. A priming was performed by adding APTES (3.4 mM) produced as described below and stirring the reaction mixture for 10 min at 20°C, 400 rpm. Apyrase (4.5 mg/mL, 28 µM) was added, and the reaction mixture was allowed to react for 10 min at 20°C, 400 rpm. An organosilica layer was grown at the surface of the immobilized apyrase using APTES (7.5 mM) and TEOS (79 mM). The resulting suspension was allowed to react for 5 hours at 20°C, 400 rpm. The particles were centrifuged for 5 min at 20000 rcf and washed 3 times in H₂O/PS80 (8 mg/L). SNP-apyrase-AT were cured overnight in a water bath at 20°C.

### Production of SNP-apyrase-AT-Chitosan

To SNPs (10 mg/mL, 62 nm) in H₂O/PS80 (8 mg/L) was added APTES (3.4 mM). The reaction mixture was allowed to react for 10 min at 20°C, 400 rpm. Then, glutaraldehyde (3.4 mM) was added, and the reaction mixture was stirred for 10 min at 20°C, 400 rpm. A priming was performed by adding APTES (3.4 mM) and stirring the reaction mixture for 10 min at 20°C, 400 rpm. Apyrase (4.5 mg/mL, 28 µM) produced as described below was added, and the reaction mixture was allowed to react for 10 min at 20°C, 400 rpm. An organosilica layer was grown at the surface of the immobilized apyrase using APTES (7.5 mM) and TEOS (79 mM). The resulting suspension was allowed to react for 5 hours at 20°C, 400 rpm. The particles were washed 3 times (by centrifugation during 5 min at 20000 ref) in H₂O/PS80 (8 mg/L) and resuspended in H₂O/PS80 (8 mg/L). A solution of chitosan in acetic acid (0.1 M) was added to the particle suspension to achieve a final chitosan concentration of 81 µg/mL. The reaction mixture was allowed to react for 30 min at 20°C, 400 rpm. The particles were centrifuged for 5 min at 20000 rcf and washed 3 times in PBS 1X. SNP-apyrase-AT-Chitosan were cured overnight in a water bath at 20°C.

### Evaluation of the immobilization rate of apyrase: Lowry assay

The amount of enzyme immobilized was quantified using the indirect Lowry protein quantification method (i.e. by measuring the protein content of the reaction supernatant). Lowry reagent was prepared by mixing a solution (500 mL) containing NaOH (2 g) and Na₂CO₃ (10 g) with a solution (10 mL) containing potassium sodium tartrate (100 mg) and CuSO₄ (50 mg). A standard regression curve with known concentrations of protein was built using Bovine Serum Albumin (BSA). To the reaction supernatant (200 µL) was added 1 mL of the Lowry reagent. The reaction mixture was vortexed and incubated at room temperature for 15 minutes. Subsequently, 100 µL of Folin reagent 1N were added and the resulting solution was incubated for 30 minutes at room temperature. Finally, the absorbance was measured at 750 nm using Biotek Synergy H1 microplate Reader.

### Size distribution and thickness layer: Scanning Electron Microscope (SEM)

A small aliquot of particles solution in PBS 1X was centrifuged at 20000 rcf for 5 min and washed 3 times with sterile water. After each washing step, the solution was sonicated for 5 minutes in a bath sonicator. Particle size measurement was carried out on SEM micrographs acquired at a magnification of 150 000x using the image analysis software Olympus Stream Motion. The ferret diameter of particles was evaluated using the software Image J.

### Apyrase enzymatic activity assay

Enzymatic activities of the produced apyrase-based nanoparticles were assessed using a colorimetric ATPase Assay Kit (Abcam), following the manufacturer's protocol. In summary, 20 µL of diluted samples (prepared in Assay Buffer) were added to a reaction mixture containing Assay Buffer and ATPase substrate and incubated at 37°C for 30 minutes. Subsequently, 30 µL of ATPase Developer were added to the reaction mixture, followed by an additional incubation at 37°C for 30 minutes. Absorbance was measured at 650 nm using a Biotek Synergy H1 microplate reader.

### Production of recombinant apyrase

For production of recombinant apyrase (SEQ ID NO: 1), the apyrase gene from *Shigella flexneri* was cloned into the pET21a vector (Novagen 69740) with a C-terminal histidine tag. The construct was codon optimized for expression in *E. coli* by Genescript. The plasmid was transformed into Rosetta (DE3)pLysS cells (Novagen 70956) and plated on LB-agar (Sigma L2897) supplemented with 100 µg/ml ampicillin and 34 µg/ml chloramphenicol. After overnight incubation, a single colony was picked and inoculated into 50 ml of LB medium (Sigma L3032) supplemented with 100 µg/ml ampicillin and 34 µg/ml chloramphenicol. The culture was grown overnight to prepare the starter culture. For purification of apyrase, induction of a 4 L culture was performed by adding 0.5 mM isopropyl-1-thio-β-d-galactopyranoside (IPTG, Sigma 16758). The culture was harvested by centrifugation at 6000 rpm for 10 minutes at 4 °C (Fiberlite^{™} F9-6 x 1000, Thermo Scientific) and the resulting cell pellet washed in 10 mM Tris pH 8.0, 150 mM NaCl, 1 mM EDTA. The cell pellet was then resuspended in 160 ml of 50 mM Tris-HCl, pH 7.5, and snap frozen in liquid nitrogen. The frozen cell pellet was thawed by immersion in room temperature water and supplemented with 300 mM NaCl and 40 mM imidazole (Sigma I202). The cell suspension was sonicated and clarified by centrifugation at 15000 rpm for 45 minutes at 4 °C (Fiberlite^{™} F20-12 x 50 LEX, Thermo Scientific). The clarified lysate was passed through a column (Pierce^{™} Disposable Columns, 10 ml, Thermo Scientific) containing 4 ml of Ni-NTA agarose beads (Qiagen) by gravity flow. The column was then washed twice with 30 ml of lysis buffer (50 mM Tris-HCl, pH 7.5, 300 mM NaCl, and 40 mM imidazole). The bound protein was eluted in 1 ml fractions with the addition of 20 ml of elution buffer (50 mM Tris-HCl, pH 7.5, 300 mM NaCl, and 300 mM imidazole). Aliquots of the collected fractions were loaded onto an SDS-PAGE gel and stained with Imperial Protein Staining (Thermo Scientific) following the manufacturer's instructions to determine the amount and purity of the enzyme. Fractions containing apyrase were pooled and concentrated to 5 ml using a 10-kDa MWCO spin concentrator (Amicon ultra-15, Millipore) and loaded onto a Hi load s-75 size exclusion column (Cytiva) pre-equilibrated with phosphate-buffered saline (PBS, Sigma D8537). The peak fractions were pooled and concentrated to approximately 2 mg/ml using a spin concentrator. The purified protein was filter sterilized using a 0.22 µm Millex^{®}-GP filter (Millipore) and stored in aliquots under appropriate conditions.

### Dextran sodium sulfate-induced colitis

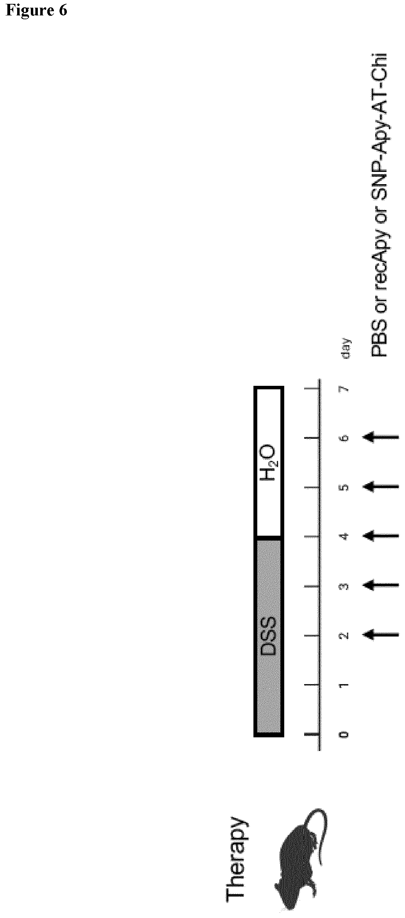

Ten-week-old mice were divided in 5 experimental groups. The control group was not treated and in the other 4 groups we administered 2% of DSS in drinking water for 4 days. Starting from day 4, we replaced 2% DSS drinking water with normal drinking water until day 7. Mice were treated with the drug daily from day 2 to day 6, as indicated in Figure 6. One group was orally gavaged daily with PBS, one group daily with recombinant apyrase at 2 mg/kg (40 µg, 22 mU), another group with the SNP-Apyrase-AT-Chitosan at 1.25 mg/kg (25 µg, 2.2 mU). To measure individual weight variations over time, ear notching was performed to every mouse at day -5 before the start of the experiment concomitantly with initial weight determination. After euthanasia, blood, faeces and large intestine was collected for analysis.

### Collection of biosamples

For *ex vivo* experiments, mice were euthanized by CO₂ inhalation, blood was collected directly from the heart using a syringe with a 26G needle, left for one hour at room temperature and serum was separated by centrifugation (twice for 10 min at maximum speed) and stored at -80°C for further analysis. Afterwards, the abdomen was carefully opened, and the colon was harvested. The colon length was measured using a standard ruler (0.1 cm interval).

### Fluorescein isothiocyanate-dextran (FITC)-dextran assay

Intestinal permeability was assessed using FITC-dextran assay. Mice were starved for 4h before treatment and then orally gavaged with 6 mg/mouse FITC-dextran (MW 4000 Da) (46944, Sigma Aldrich, Sweden) in PBS (D8537, Sigma Aldrich, UK). Blood was collected 4 h post-treatment, the serum samples were diluted 1:2 with PBS 25 mM HEPES (15630-056, Gibco, UK) and the fluorescence intensity was measured in the serum at 485/530 nm using a micro-plate reader (Biotek Synergy 2).

### Results

### Example 1: Production and characterization of SNP-apyrase-AT

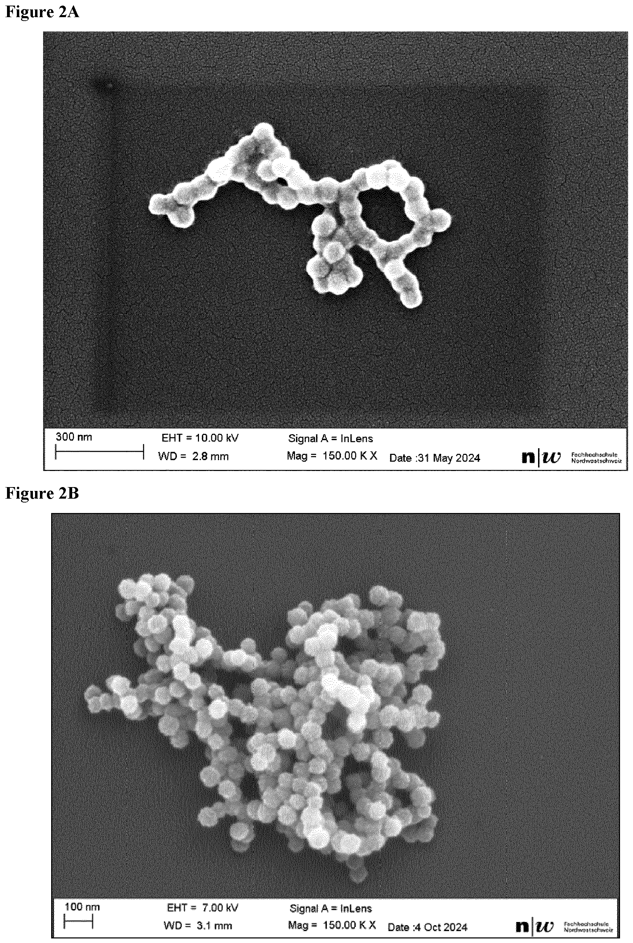

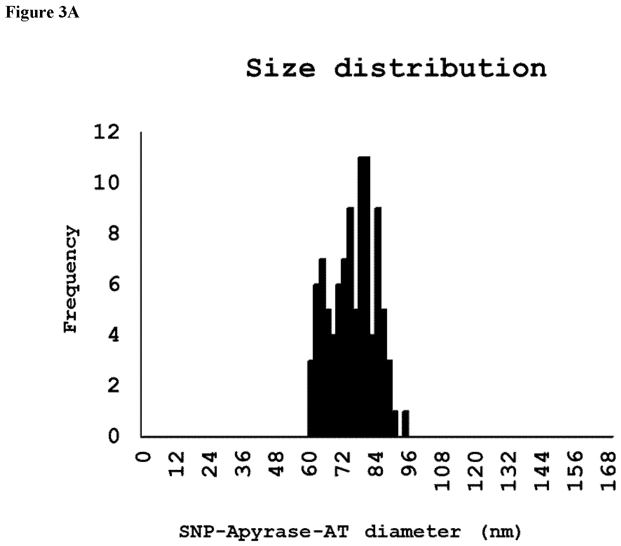

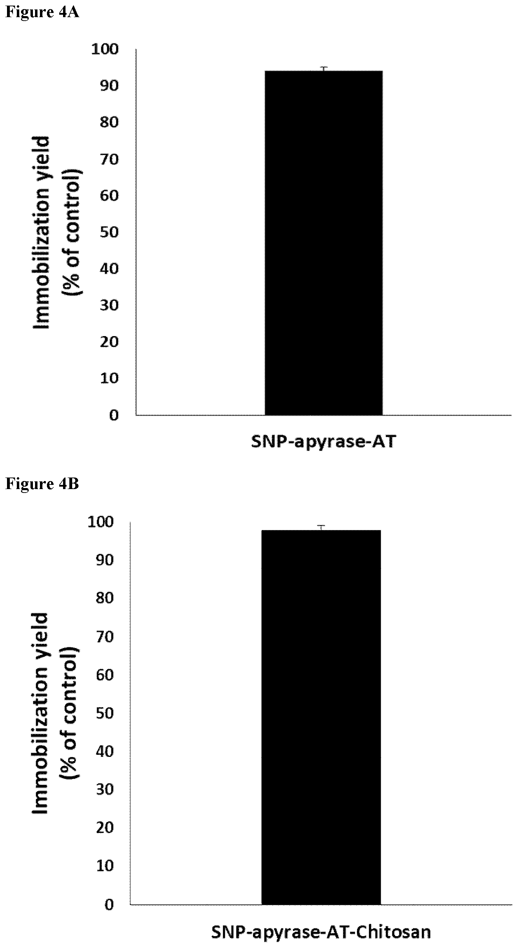

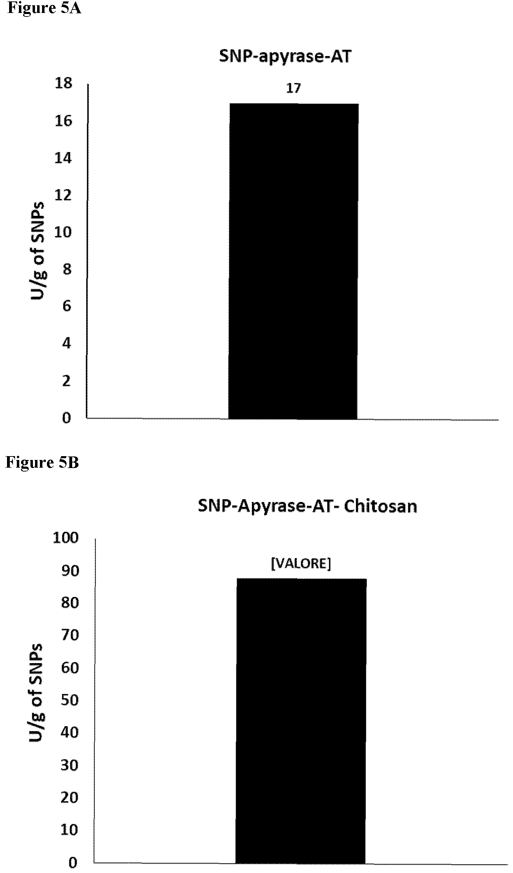

With the successful production of SNP-apyrase-AT as described above, a 14.5 mg/mL homogeneous solution of nanoparticles was obtained. Scanning electron microscopy (SEM) imaging revealed a spherical morphology of the SNPs (Figure 2A). From these images, an average particle diameter of 74 ± 8 nm was calculated, with a monodisperse size distribution (Figure 3A) and a thickness layer of 8 nm. The Lowry assay, which estimates protein content in solution, demonstrated an apyrase immobilization efficiency of 94% on SNP-apyrase-AT, relative to the total amount used for immobilization. (Figure 4A). Further analysis by ATPase assay kit confirmed the efficacy of SNP-apyrase-AT, indicating an enzymatic activity of 17 U/g of SNP, meaning 1 g of SNPs produces 17 µmol of ATP per minute (Figure 5A).

### Example 2: Production and characterization of SNP-apyrase-AT-Chitosan

A novel batch of SNP-apyrase-AT functionalized with chitosan (SNP-apyrase-AT-Chitosan) was synthesized as described above. Chitosan functionalization provided muco-adhesive properties to the nanoparticles. The resulting nanoparticles exhibited a concentration of 14.5 mg/mL and a spherical morphology (Figure 2B).

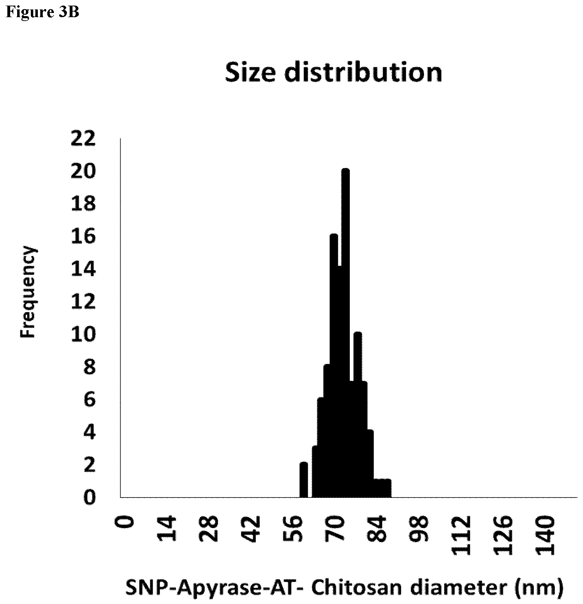

The average particle diameter was 73 ± 5 nm, with a monodisperse size distribution (Figure 3B) and a thickness layer of 7 nm. Apyrase immobilization efficiency was calculated at 98% of the total enzyme used (Figure 4B). Enzymatic activity, assessed using an ATPase assay kit, was determined to be 87.7 U/g of SNPs (Figure 5B), representing an improvement over SNP-apyrase-AT. This enhancement was achieved by resuspending and curing the nanoparticles in PBS 1X, as described in the Materials and Methods section.

### Example 3: Enhanced therapeutic protection from colitis in DSS induced IBD by SNP-apyrase-AT-Chitosan

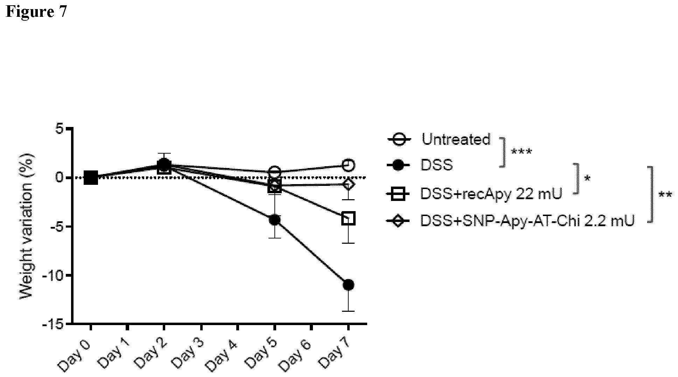

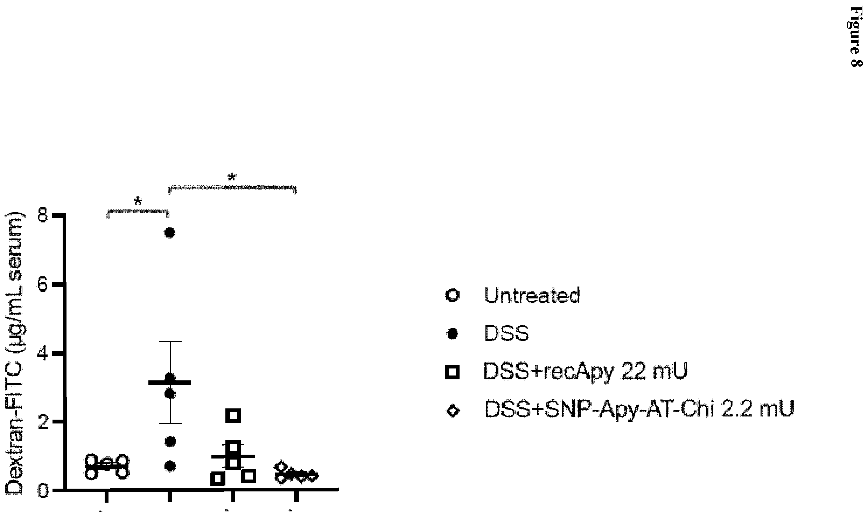

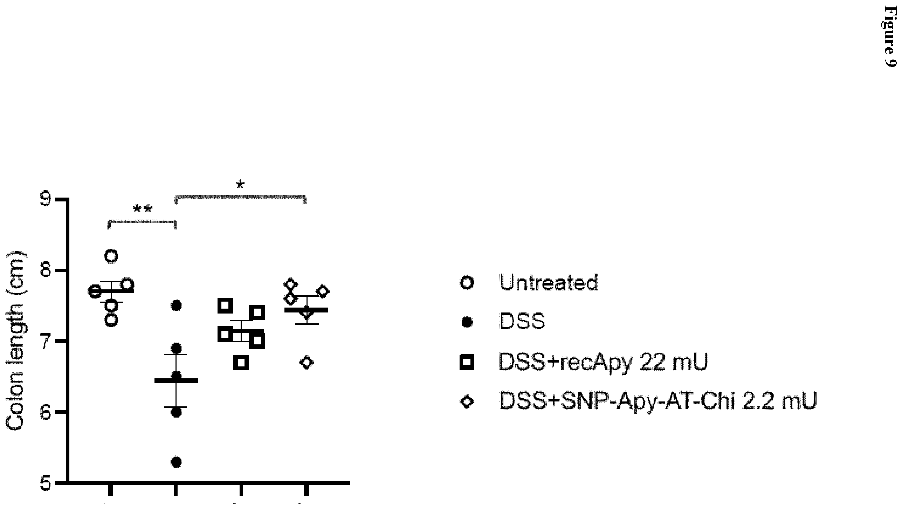

Purified recombinant apyrase from Shigella flexneri and SNP-apyrase-AT-Chitosan both produced as described above were administered to 10-week-old C57Bl/6 mice at a daily oral dosage of 2 mg/kg (40 µg, 22 mU) and 1.25 mg/kg (25 µg, 2.2 mU), respectively, in the DSS model of the disease as shown in Figure 6. To evaluate the efficacy of the treatment, we considered weight loss (Figure 7), permeability to FITC-dextran (Figure 8) and reduced colon length (Figure 9) as parameters dependent on colitis severity. Surprisingly, 10 fold reduction in apyrase enzymatic activity of SNP-apyrase-AT-Chitosan resulted in superior improvement of body weight with respect to mice treated with recombinant apyrase. Mice treated with DSS and PBS showed variable levels of increased fluorescence in the serum after the post-treatment recovery phase, indicating variable degrees of impairment of the intestinal barrier integrity at this stage. Conversely, treatment with SNP-apyrase-AT-Chitosan, but not recombinant apyrase, resulted in significantly lower and homogeneous levels of serum fluorescence with respect to PBS treated mice. Mice treated with SNP-apyrase-AT-Chitosan, but not recombinant apyrase, showed colon length values after the post-treatment recovery phase that were significantly higher than colitic mice treated with PBS. Altogether, these results demonstrate the surprising efficacy of SNP-apyrase-AT-Chitosan at low dosage in controlling the pathological consequences of colonic inflammation.

## Claims

1. A composition comprising a solid carrier, an ATP-hydrolyzing enzyme or a fragment thereof immobilized on the surface of the solid carrier, a protective layer to protect the ATP-hydrolyzing enzyme or the fragment thereof by embedding the ATP-hydrolyzing enzyme or the fragment thereof, and a functional constituent immobilized on the surface of the protective layer, wherein the functional constituent immobilized on the surface of the protective layer is a polymer comprising repeat units wherein each repeat unit comprises at least one amino group and/or at least one thiol group.

2. The composition according to claim 1, wherein the polymer comprising repeat units wherein each repeat unit comprises at least one amino group and/or at least one thiol group is a polyglucosamin selected from the group consisting of chitin, chitosan, polyglucosaminoglycans, chondroitin, heparin, keratan and dermatan or a derivative thereof.

3. The composition according to anyone of claims 1-2, wherein the polymer comprising repeat units wherein each repeat unit comprises at least one amino group and/or at least one thiol group is chitosan or a derivative thereof.

4. The composition according to anyone of claims 1-3, wherein the ATP-hydrolyzing enzyme or a fragment thereof is selected from the group consisting of apyrase or a fragment thereof, ATPase or a fragment thereof, ATP-diphosphatase or a fragment thereof, adenosine diphosphatase or a fragment thereof, ADPase or a fragment thereof, ATP-diphosphohydrolase or a fragment thereof and CD39 (Ectonucleoside triphosphate diphosphohydrolase 1, ENTPD 1) or a fragment thereof.

5. The composition according to anyone of claims 1-3, wherein the ATP-hydrolyzing enzyme or a fragment thereof is apyrase or a fragment thereof.

6. The composition according to claim 5, wherein the apyrase or a fragment thereof is a recombinant apyrase or a fragment thereof.

7. The composition according to claim 6, wherein the recombinant apyrase or a fragment thereof comprises the amino acid sequence as shown in SEQ ID NO: 1 or SEQ ID NO: 2.

8. The composition of anyone of claims 1-7, for use as a medicament.

9. The composition of anyone of claims 1-7, for use in a method for the prevention, delay of progression or treatment of dysbiosis or a dysbiosis-related disease.

10. The composition for use of claim 9, wherein dysbiosis or the dysbiosis-related disease is selected from inflammatory diseases, gastrointestinal tract-related disorders, metabolic disorders, CNS-related disorders, cancers and autoimmune diseases.

11. The composition for use of claim 9, wherein dysbiosis or a dysbiosis-related disease is selected from the group consisting of ulcerative colitis, Crohn's disease and Graft-versus-host disease.

12. A pharmaceutical combination comprising
(i) the composition of anyone of claims 1-7; and
(ii) an immune checkpoint modulator.

13. The pharmaceutical combination of claim 12 for use in a method for the prevention, delay of progression or treatment of cancer and/or for use in adoptive (T) cell therapy,

14. A method of producing a composition, the composition comprising a solid carrier, an ATP-hydrolyzing enzyme or a fragment thereof immobilized on the surface of the solid carrier, a protective layer to protect the ATP-hydrolyzing enzyme or the fragment thereof by embedding the ATP-hydrolyzing enzyme or the fragment thereof, and a functional constituent immobilized on the surface of the protective layer, wherein the functional constituent immobilized on the surface of the protective layer is a polymer comprising repeat units, wherein each repeat unit comprises at least one amino group and/or at least one thiol group, the method comprising the following steps:
(a) providing a solid carrier;
(b) immobilizing a ATP-hydrolyzing enzyme or a fragment thereof on the solid carrier;
(c) forming a protective layer on the surface of the solid carrier to protect the ATP-hydrolyzing enzyme or the fragment thereof immobilized on the solid carrier;
(d) immobilizing a functional constituent on the surface of the protective layer, wherein the functional constituent immobilized on the surface of the protective layer is a polymer comprising repeat units, wherein each repeat unit comprises at least one amino group and/or at least one thiol group.

15. A composition comprising a solid carrier, an ATP-hydrolyzing enzyme or a fragment thereof immobilized on the surface of the solid carrier, a protective layer to protect the ATP-hydrolyzing enzyme or the fragment thereof by embedding the ATP-hydrolyzing enzyme or the fragment thereof, and a functional constituent immobilized on the surface of the protective layer, wherein the functional constituent immobilized on the surface of the protective layer is a polymer comprising repeat units, wherein each repeat unit comprises at least one amino group and/or at least one thiol group, wherein the composition is obtainable by the method of claim 14.
